# EUROPEAN PATENT SPECIFICATION

(11) **EP 3 170 002 B1**
(45) Date of publication and mention of the grant of the patent: **30.12.2020**
(21) Application number: 15822023.6
(22) Date of filing: 10.07.2015
(51) Int. Cl.: G01N 33/52, G01N 31/22, G01N 33/543, G01N 33/531, G01N 27/12, B82Y 30/00

(54) **FORMULATIONS FOR ENHANCED CHEMIRESISTIVE SENSING**
FORMULIERUNGEN ZUR VERBESSERTEN CHEMIRESISTIVEN MESSUNG
FORMULATIONS PERMETTANT D'AMÉLIORER LA DÉTECTION CHIMIORÉSISTIVE

(30) Priority: 15.07.2014 US 201462024924 P
(43) Date of publication of application: 24.05.2017
(73) Proprietor: C2Sense, Inc., Cambridge, MA 02138 (US)
(72) Inventor: SCHNORR, Jan, Markus, Cambridge, MA 02138 (US); ZENTNER, Cassandra, Aileen, Brighton, MA 02135 (US); PETTY, Alexander, Robertson, Boston, MA 02134 (US); SWAGER, Timothy, Manning, Newton, MA 02458 (US)
(74) Representative: Hoffmann Eitle
(86) International application number: PCT/US2015/039971
(87) International publication number: WO 2016/010855

(56) References cited:
- WO-A1-2014/068554
- WO-A1-2015/035243
- WO-A2-2009/136978
- WO-A2-2012/119994
- WO-A2-2013/009875
- CN-A- 102 645 476
- CN-A- 103 412 026
- CN-U- 202 994 717
- US-A1- 2003 108 785
- US-A1- 2008 000 290
- US-A1- 2008 094 078
- US-A1- 2009 293 590
- US-A1- 2011 237 000
- US-A1- 2012 181 173
- US-A1- 2013 096 030
- US-A1- 2014 105 242
- JOSEPH M. AZZARELLI ET AL: "Wireless gas detection with a smartphone via rf communication", PROCEEDINGS NATIONAL ACADEMY OF SCIENCES PNAS, vol. 111, no. 51, 8 December 2014 (2014-12-08), pages 18162-18166, XP055385055, US ISSN: 0027-8424, DOI: 10.1073/pnas.1415403111
- MELTEM TUNCKOL ET AL: "Carbon nanomaterialionic liquid hybrids", CARBON, ELSEVIER, OXFORD, GB, vol. 50, no. 12, 10 May 2012 (2012-05-10), pages 4303-4334, XP028403893, ISSN: 0008-6223, DOI: 10.1016/J.CARBON.2012.05.017 [retrieved on 2012-05-18]
- XIAOWEI YU ET AL: "[beta]-cyclodextrin non-covalently modified ionic liquid-based carbon paste electrode as a novel voltammetric sensor for specific detection of bisphenol A", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, vol. 186, 1 September 2013 (2013-09-01), pages 648-656, XP055328970, NL ISSN: 0925-4005, DOI: 10.1016/j.snb.2013.06.089
- SOMAYEH KHEZRIAN ET AL: "Label-free electrochemical IgE aptasensor based on covalent attachment of aptamer onto multiwalled carbon nanotubes/ionic liquid/chitosan nanocomposite modified electrode", BIOSENSORS AND BIOELECTRONICS, vol. 43, 1 May 2013 (2013-05-01), pages 218-225, XP055430661, NL ISSN: 0956-5663, DOI: 10.1016/j.bios.2012.12.006

## Description

### Background

Gas sensing technology is being used in a wide variety of applications such as safety, security, process monitoring or air quality control. Additional applications such as ethylene or biogenic amine sensing in the food industry could benefit from gas sensors; however, current sensor technology cannot meet the necessary requirements.

Current methods for detecting ethylene, biogenic amines, or ammonia in particular include gas chromatography, photoacoustic spectroscopy, as well as electrochemical and colorimetric methods. Each of these methods has disadvantages and trade-offs, including being cumbersome, expensive, complex, bulky, limited in sensitivity and selectivity, lacking in accuracy, and requiring significant power for operation and/or highly trained technicians to carry out analysis.

Chemiresistive sensors have the potential of overcoming many of these limitations and lead to sensing technology that is scalable, multiplexed, low-cost, low-power, portable, highly selective, and highly sensitive. In order to become a feasible technology for real-world applications, these sensors need to be sufficiently selective and have a sufficiently large response to the desired analyte.

The formation of a paste of SWCNTs and ionic liquids without including detector molecules and without mentioning the use in sensing was previously described by Fukushima *et al.* (Fukushima et al., Science, 300, 2072-2074 and US 7,531,114 B2).

WO 2015/035243; J. M. Azzarelli et al (2014); M. Tunckhol et al (2012); CN 202 994 717; CN 102 645 476; X. Yu et al (2013); and S. Kherian et al (2013) disclose sensing devices.

### Summary

One method to improve the sensing response is disclosed. A sensor material including: a plurality of conductive carbonaceous nanomaterial particles, which are carbon nanotubes; a detector capable of interaction with an analyte of interest, the detector being an organic, an organometallic or an inorganic species, and the detector comprising a functional group capable of binding an analyte of interest in a solution, vapor phase or solid phase; and an ionic liquid, wherein the plurality of carbon nanotubes, the detector and the ionic liquid are combined to form a paste, wherein the ionic liquid is selected to facilitate analyte interaction with the paste resulting in a change of the conductivity of the paste, and wherein the carbon nanotubes are mixed with the detector in a ratio ranging from 3:1 to 1:10 by weight is described.

In any of the preceding embodiments, the ionic liquid includes cations selected from the group consisting of imidazolium cations, pyridinium cations, pyrrolidinium cations, phosphonium cations, and combinations thereof. In some other embodiments, the ionic liquid includes an anion selected from the group consisting of bis(trifluoromethanesulfonyl)imide (TFSI-) anions, bis(fluorosulfonyl)imide (FSI-) anions, halide anions, nitrate anions, tetrafluoroborate anions, hexafluorophosphate anions, bistriflimide anions, triflate anions, tosylate anions and combinations thereof. In some other embodiments, the ionic liquid includes non-halogenated organic anions selected from a group consisting of formate, alkylsulfate, alkylphosphate, glycolate and combinations thereof. In some other embodiments, the ionic liquid is 1-butyl-3-methylimidazolium tetrafluoroborate, 1-butyl-3-methylimidazolium hexafluorophosphate, 1 -ethyl-3-methylimidazolium bis(trifluoromethylsulfonyl)imide, 1 -Butyl-3-methylimidazolium bis(trifluoromethylsulfonyl)imide, or 1 -hexyl-3-methylimidazolium bis(trifluoromethylsulfonyl)imide.

In any of the preceding embodiments, the detector is covalently bonded to the carbonaceous nanomaterial particle. In some other embodiments, wherein the detector is non-covalently bonded to the carbonaceous nanomaterial particle. In some other embodiments, the detector is constricted inside the carbonaceous nanomaterial particles.

In any of the preceding embodiments, the detector is a small molecule, a polymer, or a biological species. In some other embodiments, the biological species comprises a peptide, protein, DNA, RNA or PNA.

In the preceding embodiments, the detector comprises a functional group capable of binding an analyte of interest in a solution, vapor phase, or solid phase. In some of the embodiments, the functional group is selected from a group consisting of a thiol, an aldehyde, an ester, a carboxylic acid, a hydroxyl group or combinations thereof.

In any of the preceding embodiments, the detector is electron-rich or electron-poor moiety; wherein interaction between an analyte of interest and the detector comprises an electrostatic interaction.

In any of the preceding embodiments, the detector includes a metal or metal containing compound. In some embodiments, the interaction between an analyte of interest and the detector includes binding to the metal or metal-containing compound. In some other embodiments, the metal containing compound is selected from a group consisting of titanium salts, silver salts, platinum salts, gold salts, aluminum salts, nickel salts, palladium salts, and copper salts. In some other embodiments, the metal-containing species includes a copper salt. In some other embodiments, the metal-containing species comprises a palladium salt.

In some embodiments, the detector is selected from the group consisting of PdCl₂, 5,10,15,20-tetraphenylporphyrinatocobalt(III) perchlorate ([Co(tpp)]ClO₄), 3,6-Di-2-pyridyl-1,2,4,5-tetrazine and combinations thereof.

The carbonaceous nanomaterial particles (carbon nanotubes) are mixed with the detector in a ratio ranging from 3:1 to 1:10 by weight. In some embodiments, the carbonaceous nanomaterial particles are mixed with the detector in a ratio ranging from 1:1 to 1:10 by weight.

In any of the preceding embodiments, about 0.1 to 20 weight% of the carbonaceous nanomaterial particles are mixed with the ionic liquid. In some embodiments, about 0.25 to 10 weight% of the carbonaceous nanomaterial particles are mixed with the ionic liquid.

In any of the preceding embodiments, the sensor material further includes viscosity modifier additives. In some embodiments, the viscosity modifier additive is selected from a group consisting of low molecular weight solvents, high molecular weight solvents, plasticizers, ethylene glycol, tetraethylene glycol, thinners, and mineral oils.

In an aspect a device includes a first electrode and a second electrode; a sensor material disposed in electrical contact with the first and second electrode; wherein, the sensor material includes the sensor material in accordance with any of the embodiments disclosed above.

In some embodiments, the device further includes an electrical circuit in connection with an ammeter or voltmeter to detect the change in conductivity of the paste forming the sensor material.

In some embodiments, the first and second electrodes are located on a rigid substrate. In some other embodiments, the rigid substrate is selected from glass, polymeric material and printed circuit board

In some embodiments, the first and second electrodes are located on a flexible substrate. In some other embodiments, the flexible substrate is selected from paper and a polymeric material.

In some embodiments, the first electrode and the second electrode are part of a complex circuit. In some other embodiments, the complex circuit is a Near Field Communication (NFC) chip or radio-frequency identification (RFID) chip.

In an aspect, a method of detecting an analyte includes providing a sensing device in accordance with any of the embodiments disclosed above; exposing the sensor material to an environment, wherein a change in the conductivity of the sensor material indicates the presence of the analyte; and detecting said change in conductivity of the sensor material.

In some embodiments, the method further includes transmitting the detected changes in conductivity wirelessly to another device for analysis and storage. In some embodiments, the method further includes detecting the analyte through a wireless radio frequency communication. In some embodiments, the method further includes detecting an output from a radio frequency identification tag including the sensor.

In some embodiments, the analyte is a vapor.

In some embodiments, the analyte is selected from a group consisting of a thiol, an ester, an aldehyde, an alcohol, an ether, an alkene, an alkyne, a ketone, an acid, a base, and a combination thereof.

In some embodiments, the analyte is a mold.

In some embodiments, the analyte is ethylene, a nitrogen-containing gas, or an amine.

In some embodiments, the analyte is putrescine or cadaverine.

In some embodiments, the concentration of the analyte is in the range of 0 to 10%, 0 to 5%, 0 to 1%, 0 to 1000 ppm, 0 to 100 ppm, 0 to 80 ppm, 0 to 50 ppm, 0 to 10 ppm, 0 to 5ppm, 0 to 1 ppm, 0 to 0.5 ppm, 0 to 100 ppb, 0 to 50 ppb, or 0 to 10 ppb.

In some embodiments, the sensor material further undergoes a volumetric change upon interaction with the analyte; and the method includes detecting the volumetric change and deriving information regarding the analyte from said volumetric change.

In some embodiments, the sensor material further undergoes a color change upon interaction with the analyte; and the method includes detecting the color change and deriving information regarding the analyte from said color change.

In some embodiments, the analyte interacts with the detector to form a Van der Waals interaction, a covalent bond, ionic bond, hydrogen bond, or dative bond.

In some embodiments, the analyte interacts with the detector via a binding event between pairs of biological molecules, wherein the biological molecules are proteins, nucleic acids, glycoproteins, carbohydrates, or hormones. In some other embodiments, the pair of biological molecules are selected from a group consisting of an antibody/peptide pair, an antibody/antigen pair, an antibody fragment/antigen pair, an antibody/antigen fragment pair, an antibody fragment/antigen fragment pair, an antibody/hapten pair, an enzyme/substrate pair, an enzyme/inhibitor pair, an enzyme/cofactor pair, a protein/substrate pair, a nucleic acid/nucleic acid pair, a protein/nucleic acid pair, a peptide/peptide pair, a protein/protein pair, a small molecule/protein pair, a glutathione/GST pair, an anti-GFP/GFP fusion protein pair, a Myc/Max pair, a maltose/maltose binding protein pair, a carbohydrate/protein pair, a carbohydrate derivative/protein pair, a metal binding tag/metal/chelate, a peptide tag/metal ion-metal chelate pair, a peptide/NTA pair, a lectin/carbohydrate pair, a receptor/hormone pair, a receptor/effector pair, a complementary nucleic acid/nucleic acid pair, a ligand/cell surface receptor pair, a virus/ligand pair, a Protein A/antibody pair, a Protein G/antibody pair, a Protein L/antibody pair, an Fc receptor/antibody pair, a biotin/avidin pair, a biotin/streptavidin pair, a drug/target pair, a zinc finger/nucleic acid pair, a small molecule/peptide pair, a small molecule/protein pair, a small molecule/target pair, a carbohydrate/protein pair such as maltose/maltose binding protein (MBP), a small molecule/target pair, and a metal ion/chelating agent pair.

In an aspect a method of making the sensor material includes providing a plurality of conductive carbonaceous nanomaterial particles; providing a detector selected to interact with an analyte of interest; providing an ionic liquid; mixing the plurality of conductive carbonaceous nanomaterial particles, the detector and the ionic liquid to form a paste.

In an aspect the method includes a method of making the sensor material including providing a plurality of conductive carbonaceous nanomaterial particles; providing a detector selected to interact with an analyte of interest; providing an ionic liquid; providing a solvent miscible with the detector and the ionic liquid; dissolving the detector and ionic liquid in the solvent to form a mix; adding the plurality of conductive carbonaceous nanomaterial particles to the mix; and evaporating the solvent to form a paste comprising the plurality of conductive carbonaceous nanomaterial particles, detector and ionic liquid.

### Brief Description of the Drawings

The above and other objects and advantages of the present disclosure will be apparent upon consideration of the following detailed description, taken in conjunction with the accompanying drawings, in which like reference characters refer to like parts throughout.
**FIG. 1** shows a schematic for making the sensor material using the three essential ingredients according to one or more embodiments;
**FIG**. **2** shows a schematic for the device for detecting an analyte using the sensor material in accordance with this disclosure;
**FIG. 3** shows the sensing response of sensors fabricated using a paste of SWCNTs, 1-butyl-3-methylimidazolium tetrafluoroborate (BMIM) BF₄, and 5,10,15,20-tetraphenylporphyrinatocoblat(III) perchlorate ([Co(tpp)]ClO₄) with 10 wt% SWCNT content in BMIM BF₄ and an SWCNT to [Co(tpp)]ClO₄ ratio of 1:1 by mass. Arrows indicate the start of a 100 sec exposure to 40 ppm ethylene;
**FIG. 4** shows the sensing response of a sensor fabricated using a paste of SWCNTs, BMIM BF₄, and [Co(tpp)]ClO₄ with 10 wt% SWCNT content in BMIM BF₄ and a [Co(tpp)]ClO₄ to SWCNT ratio of 4:1 by mass. Arrows indicate the start of a 300 sec and 600 sec exposure to 40 ppm ethylene;
**FIG. 5** shows the sensing response of sensors fabricated using a paste of SWCNTs, BMIM BF₄, and [Co(tpp)]ClO₄ on paper with 5 wt% SWCNT content in BMIM BF₄ and a [Co(tpp)]ClO₄ to SWCNT ratio of 10:1, 5:1, and 1:1 by mass, respectively. Arrows indicate the start of a 100 sec to 40 ppm ethylene
**FIG. 6** shows the average sensing response of sensors fabricated using a suspension of SWCNTs and [Co(tpp)]ClO₄, a suspension of SWCNTs and [Co(tpp)]ClO₄ coated with BMIM BF₄, a paste of SWCNTs, BMIM BF₄, and [Co(tpp)]ClO₄, and a suspension of pristine SWCNTs to 40 ppm ethylene;
**FIG. 7** shows the percent of initial response of sensors fabricated using a suspension of SWCNTs and [Co(tpp)]ClO₄, a suspension of SWCNTs and [Co(tpp)]ClO₄ coated with BMIM BF₄, and a paste of SWCNTs, BMIM BF₄, and [Co(tpp)]ClO₄ to 40 ppm ethylene four weeks after sensor fabrication;
**FIG. 8** shows the sensing response of sensors fabricated using a paste of SWCNTs, BMIM BF₄, and [Co(tpp)]ClO₄ with 1 wt% SWCNT content in BMIM BF₄ and a [Co(tpp)]ClO₄ to SWCNT ratio of 5:1 by mass. Arrows indicate the start of a 100 sec to 40 ppm ethylene;
**FIG. 9** shows the average sensing response of sensors fabricated using a suspension of pristine SWCNTs, and sensors fabricated from a paste of SWCNTs, BMIM BF₄, and [Co(tpp)]ClO₄ to different analytes;
**FIG. 10A** shows the sensing response of sensors fabricated using a paste of SWCNTs, BMIM BF₄, and [Co(tpp)]ClO₄ with 1 wt% SWCNT content in BMIM BF₄ and a [Co(tpp)]ClO₄ to SWCNT ratio of 5:1 by mass. Arrows indicate the start of a 100 sec expousre to 1, 2 and 5 ppm ammonia;
**FIG. 10B** shows the average sensing response recorded in **FIG. 10A**;
**FIG. 11A** shows the sensing response of sensors fabricated using a paste of SWCNTs, BMIM BF₄, and [Co(tpp)]ClO₄ with 1 wt% SWCNT content in BMIM BF₄ and a [Co(tpp)]ClO₄ to SWCNT ratio of 5:1 by mass. Arrows indicate the start of a 100 sec exposure to 2, 4, and 8 ppm cadaverine;
**FIG. 11B** shows the average sensing response recorded in **FIG. 11A**;
**FIG. 12A** shows the sensing response of sensors fabricated using pastes of SWCNT, PdCl₂, and three types of ionic liquids: 1-ethyl-3-methylimidazolium bis(trifluoromethylsulfonyl)imide (Ethyl TFMS), 1-Butyl-3-methylimidazolium bis(trifluoromethylsulfonyl)imide (Butyl TFMS), 1 -Hexyl-3-methylimidazolium bis(trifluormethylsulfonyl)imide (Hexyl TFMS). The lines indicate the start and end of the 500 sec exposure to 80ppm ethylene;
**FIG. 12B** shows the average sensing response recorded in **FIG. 12A** and the average sensing response of sensors fabricated by drop-casting from a suspension of SWCNTs and PdCl₂ in isopropanol;
**FIG. 12C** shows the sensing response of sensors fabricated using pastes of SWCNT, PdCl₂, and BMIM BF₄ and sensors fabricated by drop-casting from a suspension of SWCNTs and PdCl₂ in isopropanol to 80 ppm ethylene. The lines indicate the start and end of the 500 sec exposure to 80ppm ethylene;
**FIG. 13** shows the sensing response of sensors fabricated using pastes of SWCNT, PdCl₂, and 1-butyl-3-methylimidazolium hexafluorophosphate (Butyl HFP) to 80 ppm of ethylene. The lines indicate the start and end of a 500 sec exposure to 80ppm ethylene;
**FIG. 14A** shows the sensing response of sensors fabricated using pastes of SWCNT, [Co(tpp)]ClO₄, and three types of ionic liquids: 1-ethyl-3-methylimidazolium bis(trifluoromethylsulfonyl)imide (Ethyl TFMS), 1-Butyl-3-methylimidazolium bis(trifluoromethylsulfonyl)imide (Butyl TFMS), 1 -Hexyl-3-methylimidazolium bis(trifluormethylsulfonyl)imide (Hexyl TFMS). The lines indicate the start and end of the 500 sec exposure to 80ppm ethylene;
**FIG. 14B** shows the average sensing response recorded in **FIG. 14A****;**
**FIG. 15** shows the average sensing response and error of sensors fabricated using a paste of SWCNTs, BMIM BF₄, and [Co(tpp)]ClO₄ with 1 wt% SWCNT content in BMIM BF₄ and a [Co(tpp)]ClO₄ to SWCNT ratio of 5:1 by mass. The sensing material was applied at various times during the fabrication process;
**FIG. 16** shows the sensing response of sensors fabricated using a paste of SWCNTs, BMIM BF₄, and PdCl₂ with 0.25 wt% SWCNT content in BMIM BF₄ and a PdCl₂ to SWCNT ratio of 5:1 by mass. The sensing material was applied at various times during the fabrication process. Arrows indicate the start of a 100 sec exposure to 40 ppm ethylene; and
**FIG. 17** shows the sensing response of sensors fabricated using a paste of SWCNTs, BMIM BF₄, and PdCl₂ with 1 wt% SWCNT content in BMIM BF₄ and a PdCl₂ to SWCNT ratio of 5:1 by mass. The sensing material was applied at various thicknesses. Arrows indicate the start of a 100 sec to 40 ppm ethylene.

### Detailed Description

A sensor material includes: a plurality of conductive carbonaceous nanomaterial particles which are carbon nanotubes; a detector selected to selectively interact with an analyte of interest, the detector being an organic, an organometallic or an inorganic species, and the detector comprising a functional group capable of binding an analyte of interest in a solution, vapor phase or solid phase; and an ionic liquid, wherein the plurality of carbon nanotubes, the detector and the ionic liquid are combined to form a paste, wherein the ionic liquid is selected to facilitate analyte interaction with the paste resulting in a change of the conductivity of the paste, and wherein the carbon nanotubes are mixed with the detector in a ratio ranging from 3:1 to 1:10 by weight. Further, the analyte can diffuse into the paste to interact with the detector to change the conductivity of the paste.

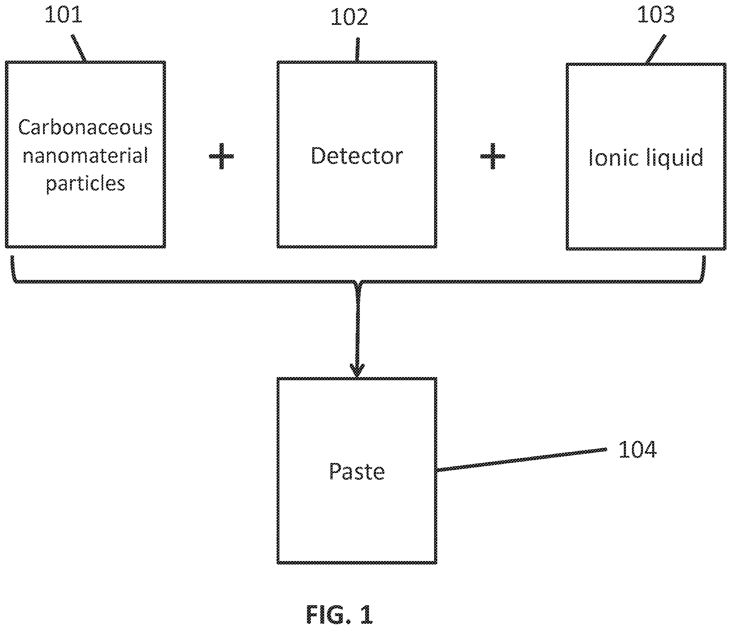

**FIG. 1** shows the components of the sensor material. The carbonaceous nanomaterial **101** is combined with the detector **102** and the ionic liquid **103** to form a paste **104.**

The carbonaceous nanomaterial particles **101** are carbon nanotubes. In some embodiments, the carbon nanotubes are single-walled nanotubes. In some embodiments, the carbon nanotubes are multi-walled nanotubes. In some embodiments, the carbon nanotubes are double-walled nanotubes.

The detector **102** is a moiety that may interact with an analyte and/or that may be responsive to a change in a surrounding medium or environment, and may be incorporated within the device in various configurations. For example, the detector **102** may be a small molecule, a polymer, a biological species, or the like. In some embodiments, the detector may comprise ionic species (e.g., a salt). In some embodiments, the detector **102** may comprise a neutral species. The detector **102** is an organic, organometallic, or an inorganic species. In certain embodiments, the detector **102** may be attached to the carbonaceous nanomaterial particles via a covalent bond. In certain embodiments, the detector **102** may be attached to the carbonaceous nanomaterial particles via a non-covalent bond. In certain other embodiments, the detector **102** may be substantially contained within (e.g., dispersed within) the carbonaceous nanomaterial particles, and may not form a covalent bond to the carbonaceous nanomaterial particles.

The detector **102** comprises a biological or a chemical group capable of binding another biological or chemical molecule in a medium (e.g., solution, vapor phase, solid phase). For example, the detector **102** may include a functional group, such as a thiol, aldehyde, ester, carboxylic acid, hydroxyl, and the like, wherein the functional group forms a bond with the analyte. In some cases, the detector **102** may be an electron-rich or electron-poor moiety wherein interaction between the analyte and the detector comprises an electrostatic interaction. In some embodiments, the interaction between the analyte and the detector **102** includes binding to a metal or metal-containing moiety.

In some embodiments, the detector **102** may be a metal-containing species. For example, the species may be a metal-containing species, including metal salts. In some embodiments, the metal salt is a transition metal salt or complex. Some examples of metal salts include, but are not limited to, TiO₂, TiCl₄, and other titanium salts, AgCl, AgPF₆, Ag(OCOCF₃), Ag(SO₃CF₃), and other silver salts, PtCl₂ and other platinum salts, Au₂Cl₆ and other gold salts, Al(OEt)₃ and other aluminum salts, Ni(SO₃CF₃)₂, NiCl₂, and other nickel salts, and Cu(SO₃CF₃) and other copper salts, In some embodiments, the species may be a copper-containing species. In some embodiments, the copper-containing species is a salt, such as a Cu(II) salt. In some embodiments, the species may be a palladium-containing species. In some embodiments, the palladium-containing species is a salt, such as a Pd(II) salt. Some examples of specific metal containing species include, but are not limited to, PdCl₂. In certain embodiment, the detector **102** includes 5,10,15,20-tetraphenylporphyrinatocoblat(III) perchlorate ([Co(tpp)]ClO₄), 3,6-Di-2-pyridyl-1,2,4,5-tetrazine and combinations thereof.

In certain embodiments, a combination of detectors described above is used in form the paste to be used as the sensor material. For example, PdCl₂ and a copper salt may be combined to be used as detector **102** for detecting ethylene.

Any of the known ionic liquids may be used as the ionic liquid **103** in accordance with the disclosure. In some other embodiments, the ionic liquid **103** includes cations selected from the group consisting of imidazolium cations, pyridinium cations, pyrrolidinium cations, phosphonium cations, and combinations thereof. In some other embodiments, the ionic liquid 103 includes an anion selected from the group consisting of bis(trifluoromethanesulfonyl)imide (TFSI-) anions, bis(fluorosulfonyl)imide (FSI-) anions, halide anions, nitrate anions, tetrafluoroborate anions, hexafluorophosphate anions, bistriflimide anions, triflate anions, tosylate anions and combinations thereof. In some other embodiments, the ionic liquid **103** includes non-halogenated organic anions selected from a group consisting of formate, alkylsulfate, alkylphosphate, glycolate and combinations thereof. In certain embodiments the ionic liquid is 1-butyl-3-methylimidazolium tetrafluoroborate, 1-butyl-3-methylimidazolium hexafluorophosphate, 1-ethyl-3- methylimidazolium bis(trifluoromethylsulfonyl)imide, 1 -Butyl-3-methylimidazolium bis(trifluoromethylsulfonyl)imide, or 1 -hexyl-3-methylimidazolium bis(trifluormethylsulfonyl)imide.

The carbonaceous nanomaterial particles (carbon nanotubes) **101** are mixed with the detector 102 in a ratio ranging from 3:1 to 1:10 by weight. In certain embodiments, the ratio of the carbonaceous nanomaterial particles **101** to detector **102** is 1:1 by weight. In certain other embodiments, the ratio of the carbonaceous nanomaterial particles **101** to detector **102** is 1:5 by weight. In certain other embodiments, the ratio of the carbonaceous nanomaterial particles **101** to detector **102** is 1:10.

In some embodiments, about 0.1 to 20 weight % of the carbonaceous nanomaterial particles **101** is mixed with the ionic liquid **103.** In some other embodiments, about 0.25 to 20 weight % of the carbonaceous nanomaterial particles **101** is mixed with the ionic liquid **103.** In some embodiments, about 1 to 20 weight % of the carbonaceous nanomaterial particles **101** is mixed with the ionic liquid **103.** In some other embodiments, about 5 to 20 weight% of the carbonaceous nanomaterial particles **101** is mixed with the ionic liquid **103.** In some other embodiments, about 10 to 20 weight % of the carbonaceous nanomaterial particles **101** is mixed with the ionic liquid **103.** In some other embodiments, about 0.25 to 10 weight % of the carbonaceous nanomaterial particles **101** is mixed with the ionic liquid **103.** In some embodiments, about 1 to 10 weight % of the carbonaceous nanomaterial particles **101** is mixed with the ionic liquid **103.** In some other embodiments, about 5 to 10 weight % of the carbonaceous nanomaterial particles **101** is mixed with the ionic liquid **103.**

In some embodiments, the carbonaceous nanomaterial particles **101** are present in the range of 0.1 weight % to 20 weight % in the paste. In some other embodiments, the carbonaceous nanomaterial particles **101** are present in the range of 0.1 weight % to 15 weight % in the paste. In some other embodiments, the carbonaceous nanomaterial particles **101** are present in the range of 1 weight % to 15 weight % in the paste. In some other embodiments, the carbonaceous nanomaterial particles **101** are present in the range of 5 weight % to 15 weight % in the paste.

In some other embodiments, the detector **102** is present in the range of 0.05 weight % to 65 weight % in the paste. In some other embodiments, the detector **102** is present in the range of 0.05 weight % to 45 weight % in the paste. In some other embodiments, the detector **102** is present in the range of 0.05 weight % to 15 weight % in the paste. In some other embodiments, the detector **102** is present in the range of 0.1 weight % to 65 weight % in the paste. In some other embodiments, the detector **102** is present in the range of 0.1 weight % to 45 weight % in the paste. In some other embodiments, the detector **102** is present in the range of 0.1 weight % to 15 weight % in the paste. In some other embodiments, the detector **102** is present in the range of 0.1 weight % to 15 weight % in the paste. In some other embodiments, the detector **102** is present in the range of 5 weight % to 65 weight % in the paste. In some other embodiments, the detector **102** is present in the range of 5 weight % to 45 weight % in the paste.

In some other embodiments, the ionic liquid **103** is present in the range of 20 weight % to 99.5 weight % in the paste. In some other embodiments, the ionic liquid **103** is present in the range of 20 weight % to 75 weight % in the paste. In some other embodiments, the ionic liquid **103** is present in the range of 20 weight % to 45 weight % in the paste. In some other embodiments, the ionic liquid **103** is present in the range of 25 weight % to 99.5 weight % in the paste. In some other embodiments, the ionic liquid **103** is present in the range of 25 weight % to 75 weight % in the paste. In some other embodiments, the ionic liquid **103** is present in the range of 25 weight % to 45 weight % in the paste. In some other embodiments, the ionic liquid **103** is present in the range of 30 weight % to 99.5 weight % in the paste. In some other embodiments, the ionic liquid **103** is present in the range of 30 weight % to 75 weight % in the paste. In some other embodiments, the ionic liquid **103** is present in the range of 30 weight % to 45 weight % in the paste.

In some embodiments, the sensor material further includes additives such as viscosity modifiers to tailor the physical properties of the paste used for various applications. Suitable viscosity modified may be viscosity enhancers or viscosity reducers. Some suitable viscosity modifiers include, but are not limited to, low and high molecular weight solvents, plasticizers, ethylene glycol, tetraethylene glycol, thinners, mineral oils, etc.

Any known technique of mixing may be used to form the paste using the components of the sensor material. In an embodiment, the mixing is done by ball milling, wherein all the components are added and milled for a prescribed duration to form a homogeneous paste. In some other embodiments, the mixing is done by ball milling, wherein some components are added and milled for a prescribed duration to form a homogeneous paste followed by addition of the remaining components and additional mixing in one or multiple steps. In some other embodiments, the mixing of the ingredients is carried out using a blender. In some other embodiments, the mixing of the ingredients is carried out using a mortar and pestle.

In an aspect, a device includes a first electrode and a second electrode; a sensor material disposed in electrical contact with the first and second electrode wherein, the sensor material includes, a plurality of conductive carbonaceous nanomaterial particles; a detector selected to selectively interact with an analyte of interest; and an ionic liquid. The plurality of conductive carbonaceous nanomaterial particles, the detector and the ionic liquid are mixed together to form a paste; and the analyte can diffuse into the paste to interact with the detector to change the conductivity of the paste. The device further includes an electrical circuit capable of detecting the changes in the conductivity of the paste to detect information regarding the analyte.

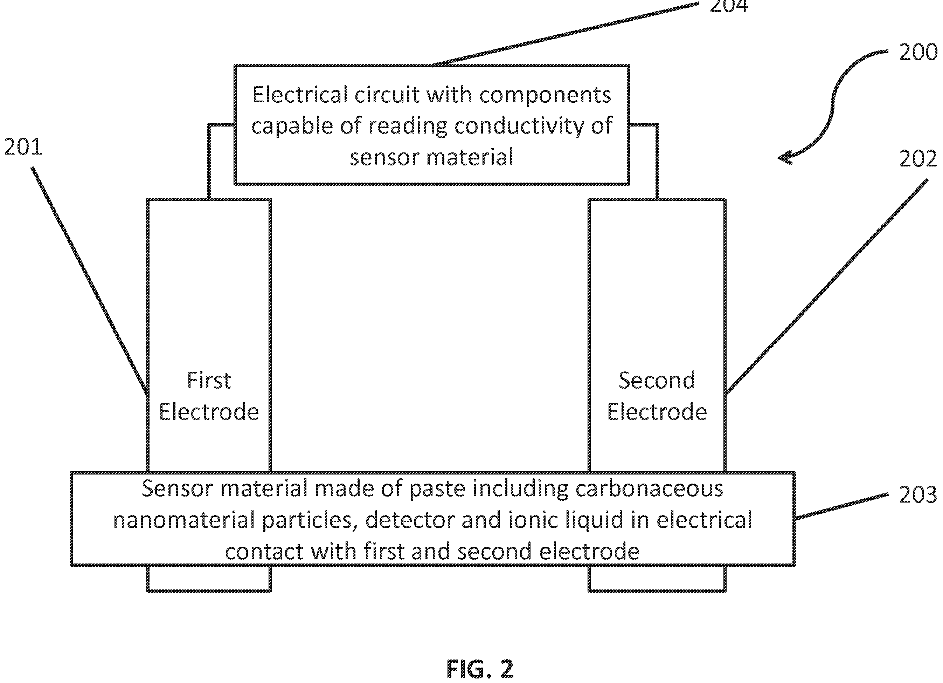

**FIG. 2** shows a schematic for the device for detecting an analyte using the sensor material in accordance with this disclosure. The device **200** includes a first electrode **201** and a second electrode **202** and a sensor material including carbonaceous nanomaterial particles, detector and ionic liquid, in electrical contact with the first electrode and the second electrode. The first electrode **201** and the second electrode **202** are connected to form an electrical circuit **204** with components capable of measuring the conductivity of the sensor material **203.**

In another aspect, the method of detecting an analyte includes providing a first electrode and a second electrode; providing a sensor material disposed in electrical contact with the first and second electrode; wherein the sensor material includes a plurality of conductive carbonaceous nanomaterial particles, a detector selected to selectively interact with an analyte of interest; an ionic liquid, wherein the plurality of conductive carbonaceous nanomaterial particles, the detector and the ionic liquid are mixed together to form a paste., The detector in the paste can interact with an analyte to change the conductivity of the sensor material. The method further includes exposing the sensor material to the analyte, wherein the exposure to analyte changes the conductivity of the sensor material, and detecting the change in conductivity of the sensor material to gather information regarding the analyte.

In some embodiments, the first and second electrodes are located on a rigid substrate such as, glass or a polymeric material. In some other embodiments, the first and second electrodes are located on a printed circuit board. In some other embodiments, the first and second electrodes are located on a flexible substrate. In some embodiments, the flexible substrate is paper. In some other embodiments, the flexible substrate is a polymeric material. In some embodiments, the first and second electrodes are printed on the flexible substrate. The printing of the electrodes may be carried out using any of the common techniques known in the art. These techniques are, but not limited to, screen printing, off-set printing, gravure printing, block printing, inkjet printing, relief printing, pad printing and intaglio.

In some embodiments , the first electrode **201** and the second electrode **202** are part of a complex circuit such as, a Near Field Communication (NFC) or radio-frequency identification (RFID) chip.

In certain embodiments, the analyte is a vapor or a gas. In some embodiments, the analyte is selected from a group consisting of a thiol, an ester, an aldehyde, an alcohol, an ether, an alkene, an alkyne, a ketone, an acid, a base, or combinations thereof. In some other embodiments, the analyte is a mold. In some other embodiments, the analyte is ethylene. In some embodiments, the analyte is a nitrogen-containing gas. In certain embodiments, the analyte is an amine. In certain embodiments, the analyte is putrescine or cadaverine.

In some embodiments, the concentration of the analyte is in the range of 0 to 10%, 10 ppm to 10%, 100 ppm to 10%,1000 ppm to 10%, 1 to 10%, or 5 to 10%. In some other embodiments, the concentration of the analyte is in the range of 0 to 5%, 10 ppm to 5%, 100 ppm to 5%, 1000 ppm to 5%, 1 to 5%, or 2 to 5%. In some other embodiments, the concentration of the analyte is in the range of 0 to 1%, 10 ppb to 1%, 100 ppb to 1%, 1 ppm to 1%, or 10 ppm to 1%. In some other embodiments, the concentration of the analyte is in the range of 0 to 1000 ppm, 10 ppb to 1000 ppm, 100 ppb to 1000 ppm, 1 ppm to 1000 ppm, or 10 ppm to 1000 ppm. In some other embodiments, the concentration of analyte is in the range of 0 to 100 ppm, 10 ppb to 100 ppm, 100 ppb to 100 ppm, 1 ppm to 100 ppm, or 10 ppm to 100 ppm. In some other embodiments the concentration of analyte is in the range of 0 to 80 ppm, 10 ppb to 80 ppm, 100 ppb to 80 ppm, 1ppm to 80 ppm, or 10 ppm to 80 ppm. In some other embodiments the concentration of analyte is in the range of 0 to 50 ppm, 10 ppb to 50 ppm, 100 ppb to 50 ppm, 1 ppm to 50 ppm, or 10 ppm to 50 ppm. In some other embodiments the concentration of analyte is in the range of 0 to 10 ppm, 10 ppb to 10 ppm, 100 ppb to 10 ppm, or 1 ppm to 10 ppm. In some embodiments, the concentration of the analyte is in the range of 0 to 1 ppm, 10 ppb to 1 ppm, or 100 ppb to 1 ppm. In some other embodiments, the concentration of the analyte is in the range of 0 to 0.5 ppm, 10 ppb to 0.5 ppm, or 100 ppb to 0.5 ppm. In some other embodiments, the concentration of the analyte is in the range of 0 to 100 ppb, or 10 ppb to 100 ppb. In some other embodiments, the concentration of the analyte is in the range of 0 to 50 ppb, or 10 ppb to 50 ppb. In some other embodiments, the concentration of the analyte is in the range of 0 to 10 ppb.

The interaction between the analyte and the detector 102 in the paste 104 used to form the sensor material 203 may include formation of a bond, such as a covalent bond (e.g. carbon-carbon, carbon-oxygen, oxygen-silicon, sulfur-sulfur, phosphorus-nitrogen, carbon-nitrogen, metal-oxygen or other covalent bonds), an ionic bond, a hydrogen bond (e.g., between hydroxyl, amine, carboxyl, thiol and/or similar functional groups, for example), a dative bond (e.g. complexation or chelation between metal ions and monodentate or multidentate ligands), and the like. The interaction may also comprise Van der Waals interactions. In one embodiment, the interaction comprises forming a covalent bond with an analyte. In some cases, the interaction between the device and the analyte may comprise a reaction, such as a charge transfer reaction. In some other embodiments, the species and/or another device component may undergo a chemical or physical transformation upon a change in the surrounding environment (e.g., change in temperature) to produce a determinable signal from the device.

The detector **102** in the paste **104** used to form the sensor material **203** may also interact with an analyte via a binding event between pairs of biological molecules including proteins, nucleic acids, glycoproteins, carbohydrates, hormones, and the like. Specific examples include an antibody/peptide pair, an antibody/antigen pair, an antibody fragment/antigen pair, an antibody/antigen fragment pair, an antibody fragment/antigen fragment pair, an antibody/hapten pair, an enzyme/substrate pair, an enzyme/inhibitor pair, an enzyme/cofactor pair, a protein/substrate pair, a nucleic acid/nucleic acid pair, a protein/nucleic acid pair, a peptide/peptide pair, a protein/protein pair, a small molecule/protein pair, a glutathione/GST pair, an anti-GFP/GFP fusion protein pair, a Myc/Max pair, a maltose/maltose binding protein pair, a carbohydrate/protein pair, a carbohydrate derivative/protein pair, a metal binding tag/metal/chelate, a peptide tag/metal ion-metal chelate pair, a peptide/NTA pair, a lectin/carbohydrate pair, a receptor/hormone pair, a receptor/effector pair, a complementary nucleic acid/nucleic acid pair, a ligand/cell surface receptor pair, a virus/ligand pair, a Protein A/antibody pair, a Protein G/antibody pair, a Protein L/antibody pair, an Fc receptor/antibody pair, a biotin/avidin pair, a biotin/streptavidin pair, a drug/target pair, a zinc finger/nucleic acid pair, a small molecule/peptide pair, a small molecule/protein pair, a small molecule/target pair, a carbohydrate/protein pair such as maltose/MBP (maltose binding protein), a small molecule/target pair, or a metal ion/chelating agent pair. Specific non-limiting examples of species include peptides, proteins, DNA, RNA, PNA.

In some embodiments, the analyte may contact, or may be positioned in sufficient proximity to the sensor material **203,** or may permeate into an interior portion of the sensor material **203** to interact with the paste **104.** In some embodiments, a volumetric or dimensional change (e.g., increase, decrease) of the sensor material may occur upon interaction with an analyte. For example, a component of the device may "swell" upon absorption of the analyte, wherein the change in volume may produce a change in a property of the device. In some embodiments, the analyte may cause a change in color of the sensor material **203.**

The applicants have surprisingly found that combination of a detector with carbonaceous nanomaterial particles and including this combination in a paste formed using ionic liquids provides unexpected and advantageous properties. These are, but not limited to, significantly improved signal of response to the presence of an analyte, elongated service life of the sensor, increased sensitivity, lower limit of detection, and enhanced selectivity. These advantageous properties cover multiple key performance metrics of a sensor and thus present a significant advance.

Applicants have further surprisingly discovered that the above paste provides greater sensitivity for sensing an analyte than compositions lacking an ionic liquid carrier and/or not in paste form. Since the analyte is required to diffuse into the paste and interact with the detector to alter the properties such as electrical properties to produce a signal which may be detected and analyzed for the detection of the analyte, one of ordinary skill in the art would expect the performance, in terms of sensitivity and selectivity, of such a sensor to be significantly reduced. Additionally, it is expected that due to the polar nature of the ionic liquids, the detector has a tendency to dissociate from the carbonaceous nanoparticle material and show a selective affinity towards the ionic liquid. Due to this the performance of the sensor can be seriously impaired. Surprisingly, and contrary to common wisdom, the applicants have identified that not only is this feasible, but the resulting sensor material provides enhanced properties as discussed below. The applicants hypothesize that the interaction of the ionic liquid with the carbonaceous nanoparticle surface leads to debundling of the carbonaceous nanoparticle and thus increases the available carbonaceous nanoparticle surface area. As a result, the detector can interact more easily with the carbonaceous nanoparticle and detector-analyte interactions lead to an enhanced effect for the conductivity of the carbonaceous nanoparticle or the network of carbonaceous nanoparticles.

Without being bound by theory, it is believed that the blending with the ionic liquid improves the dispersion of the detectors and the carbonaceous nanomaterial particles, thereby increasing the surface area of exposure. Also, contrary to the expected behavior, the presence of the ionic liquid does not impede the interaction of the analyte with the detector and the carbonaceous nanomaterial particles.

### Illustrative Examples

### Materials and Measurements

The single-walled carbon nanotubes (SWCNT), 1-butyl-3-methylimidazolium tetrafluoroborate , (97%, BMIM BF₄), 1-ethyl-3-methylimidazolium bis(trifluoromethylsulfonyl)imide (97%, Ethyl TFMS), 1-Butyl-3-methylimidazolium bis(trifluoromethylsulfonyl)imide (98%, Butyl TFMS),1-Hexyl-3-methylimidazolium bis(trifluormethylsulfonyl)imide (98%, Hexyl TFMS) with 1-Butyl-3-methylimizaolium hexafluorophosphate (97%, Butyl HFP), cadaverine (>97%), isopropanol (>99%), o-dichlorobenzene (anhydrous), Palladium (II) Chloride and 3,6-Di-2-pyridyl-1,2,4,5-tetrazine were purchased from Sigma-Aldrich and used as received. The detector, 5,10,15,20-tetraphenylporphyrinatocoblat(III) perchlorate ([Co(tpp)]ClO₄) was synthesized following literature procedures (Sugimoto et al., Bull. Chem. Soc. Jpn., 54, 3425-3432). The analytes 1% ethylene gas in nitrogen (1.0001 vol% ± 2%) and 1% ammonia gas in nitrogen (0.9979% ± 2%), and the carrier gas dry nitrogen, were obtained from AirGas.

To determine the sensing capability of the devices, the change in conductivity of the sensor was monitored upon analyte exposure. The sensing measurements were performed using a PalmSens EmStat-MUX (PalmSens BV). The devices were placed in a custom-built Teflon enclosure, consisting of an inlet/outlet for gas flow and a gas chamber for exposure of the sensor to the analyte. The device was connected to the potentiostat via a 64pin IC Test Clip (3M). A Sierra Instruments gas mixer system, a kin-tek gas generator, a custom-built setup consisting of syringe pumps, or a custom-built setup consisting of peristaltic pumps was used to generate various concentrations of the analyte by mixing the analyte with nitrogen gas, air, or humidified nitrogen gas.

Sensing material was applied to devices via two different methods 1) applied with a metal spatula or 2) using a screen printing mimicking method. The screen printing mimicking method was performed by placing a mask across the device. The mask had laser cut holes over the space between the electrodes. Sensing material was spread across the holes, and scraped using a blade giving a uniform thickness. Thickness of the material varied by using different thicknesses of the masks.

### Example 1

The following example describes the fabrication and measurement of a sensor comprised of a SWCNT-BMIM BF₄ paste, with [Co(tpp)]ClO₄ as the detector for the analyte.

The paste was prepared by grinding SWCNT, BMIM BF₄, and [Co(tpp)]ClO₄ for 10 min using mortar and pestle. The composition of the paste was 10wt% SWCNT in BMIM BF₄ and a 1:1 mass ratio of [Co(tpp)]ClO₄ to SWCNT. The substrate was made by depositing a gold electrode pattern, with a 1mm electrode gap, onto a glass slide using a thermal evaporator (Mill Lane Engineering, EV-2000). The electrode pattern was made using a shadow mask and layering 10nm of chromium then 100nm of gold. The sensors were fabricated by placing the paste between the electrodes using a metal spatula. Additional paste was added until the resistance of the sensor material between the electrodes was between 7 and 30kΩ for each sensor.

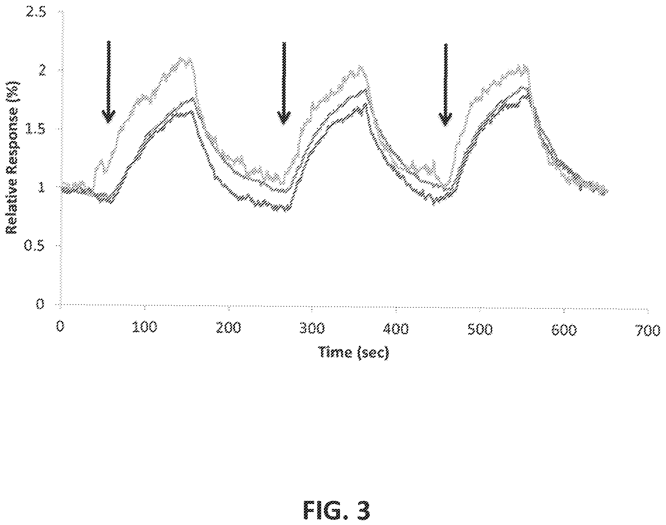

To determine the sensing capability of the devices, the change in conductivity of the sensor was monitored upon exposure to analyte, using the method described in "Materials and Measurements." The sensors were alternatingly exposed to the analyte, 40ppm ethylene in nitrogen, and the carrier gas nitrogen for three cycles. **FIG. 3** shows the sensing response of sensors fabricated using a paste of SWCNTs, BMIM BF4, and [Co(tpp)]ClO₄ with 10 wt% SWCNT content in BMIM BF4 and an SWCNT to [Co(tpp)]ClO₄ ratio of 1:1 by mass. Arrows indicate the start of a 100 sec exposure to 40 ppm ethylene. The average of the sensing response for the sensors was determined to be 0.94%.

### Example 2

The following example describes the fabrication and measurement of a sensor composed of a 3,6-di-2-pyridyl-1,2,4,5-tetrazine paste on a flexible paper device.

The paste was prepared by grinding SWCNT and BMIM BF₄ using a mortar and pestle. Then 3,6-di-2-pyridyl-1,2,4,5-tetrazine was added to achieve a 4:1 mass ratio (tetrazine:SWCNT) and the components were mixed. The substrate was made by depositing a gold electrode pattern, with a 1mm electrode gap, onto weigh paper using a thermal evaporator (Mill Lane Engineering, EV-2000). The electrode pattern was made using a shadow mask and layering 10nm of chromium then 100nm of gold. The sensor was fabricated by placing the paste between the electrodes using a metal spatula. Additional paste was added until the resistance of the sensor material between the electrodes was between 1 and 4kΩ for each sensor.

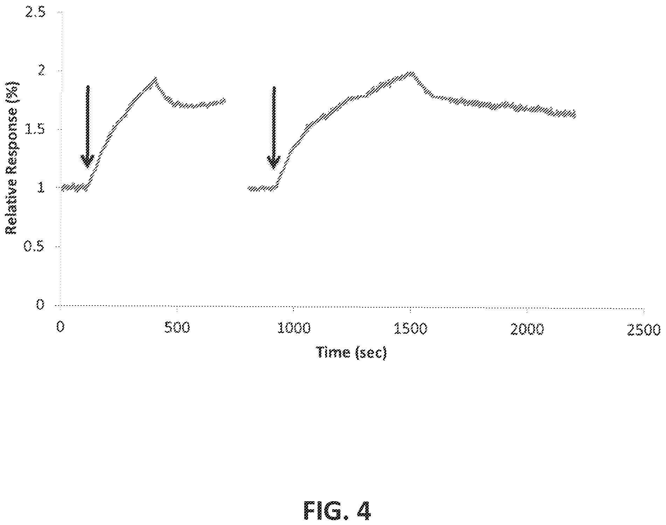

To determine the sensing capability of the devices, the change in conductivity of the sensor was monitored upon exposure to analyte, using the method described in "Materials and Measurements." The sensor was alternatingly exposed to the analyte, 40ppm ethylene in nitrogen, for 300sec and 600sec, respectively, and nitrogen gas. **FIG. 4** shows the sensing response of a sensor fabricated using a paste of SWCNTs, BMIM BF₄, and [Co(tpp)]ClO₄ with 10 wt% SWCNT content in BMIM BF₄ and a [Co(tpp)]ClO₄ to SWCNT ratio of 4:1 by mass. Arrows indicate the start of a 300 sec and 600 sec exposure to 40 ppm ethylene. The sensing response was determined to be a 0.96% with a 300sec exposure, and a 1.0% with a 600sec exposure, with a mostly irreversible response.

### Example 3

The following example describes the fabrication and measurement of [Co(tpp)]ClO₄ paste sensors on a paper device.

The pastes were prepared by grinding the SWCNT, BMIM BF₄, and [Co(tpp)]ClO₄ for 10min using an agate mortar and pestle. Three 5wt% of SWCNT in BMIM BF₄ pastes were made: 1:1, 5:1 and 10:1 mass ratio of [Co(tpp)]ClO₄ to SWCNT. The substrate was made by depositing a gold electrode pattern, with a 1mm electrode gap, onto weigh paper using a thermal evaporator (Mill Lane Engineering, EV-2000). The electrode pattern was made using a shadow mask and layering 10nm of chromium then 100nm of gold. The sensor was fabricated by placing the pastes between the electrodes using a metal spatula. Additional paste was added until the resistance of the sensor material between the electrodes was between 7 and 30kΩ for each sensor.

To determine the sensing capability of the devices, the change in conductivity of the sensor was monitored upon exposure to analyte, using the method described in "Materials and Measurements." The sensors were alternatingly exposed to the analyte, 40ppm ethylene in nitrogen, and the carrier gas nitrogen for three cycles.

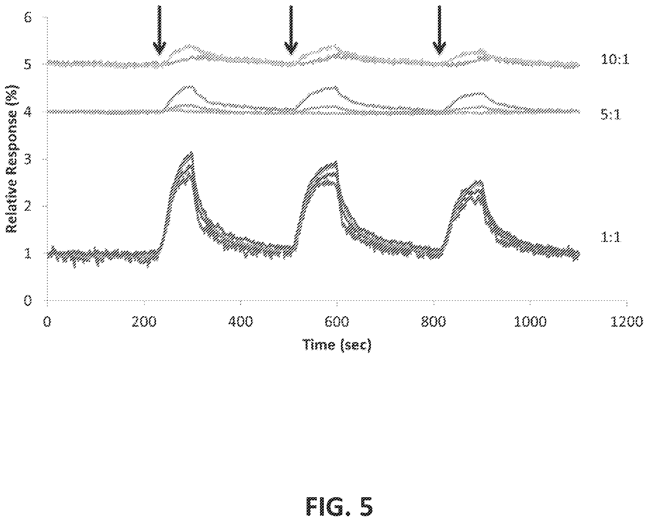

**FIG. 5** shows the sensing response of sensors fabricated using a paste of SWCNTs, BMIM BF₄, and [Co(tpp)]ClO₄ on paper with 5 wt% SWCNT content in BMIM BF₄ and a [Co(tpp)]ClO₄ to SWCNT ratio of 10:1, 5:1, and 1:1 by mass, respectively. Arrows indicate the start of a 100 sec to 40 ppm ethylene. The average sensing response for the 1:1 mass ratio pastes was 1.7%, while the 5:1 and 10:1 pastes were lower at an average of 0.24% and 0.33%, respectively.

This result suggests that as the amount of the sensor material is increased, the sensor response increases. However, a threshold is expected after which additional increase in the detector amount will not produce a proportional increase in the response, since the conductivity of the total system will be marginalized due to the reduction of the conductive carbonaceous nanomaterial particles, such as carbon nanotubes.

### Example 4

The following example describes the fabrication and measurement of three types of sensors: 1) SWCNT-[Co(tpp)]ClO₄ layer, 2) BMIM BF₄ layered on a SWCNT-[Co(tpp)]ClO₄ layer, and 3) SWCNT-[Co(tpp)]ClO₄ paste.

The substrate was made by depositing a gold electrode pattern, with a 1mm electrode gap, onto a glass slide using a thermal evaporator (Mill Lane Engineering, EV-2000). The electrode pattern was made using a shadow mask and layering 10nm of chromium then 100nm of gold.

The SWCNT-[Co(tpp)]ClO₄ sensors were prepared by dropcasting a suspension containing SWCNT and [Co(tpp)]ClO₄. The suspension was prepared by sonicating [Co(tpp)]ClO₄ (10:1 mass ratio of [Co(tpp)]ClO₄ to SWCNT) and 0.25mg/mL SWCNT in o-dichlorobenzene for 1 minute. The suspension was dropcast until the resistance of each sensor was between 7-10kΩ. Between each successive dropcast of the suspension, the device was vacuum dried until complete solvent removal.

The sensor comprised of a BMIM BF₄ layer on a SWCNT-[Co(tpp)]ClO₄ layer, was prepared by dropcasting the SWCNT-[Co(tpp)]ClO₄ suspension until the resistance of each sensor was between 2-5kΩ. Between each dropcast, the device was vacuum dried until solvent was completely removed. A 10mg/mL BMIM BF₄ solution in methanol was dropcast (1µL drop) on the SWCNT-[Co(tpp)]ClO₄ layer. The device was vacuum dried to remove the methanol.

The [Co(tpp)]ClO₄ paste sensors were prepared by grinding SWCNT, BMIM BF₄, and [Co(tpp)]ClO₄ for 20min using an agate mortar and pestle. The paste composition was 1wt% of SWCNT in BMIM BF₄ with a 5:1 mass ratio of [Co(tpp)]ClO₄ to SWCNT. The sensors were fabricated by placing the paste between the electrodes using a metal spatula. Additional paste was added until the resistance of the sensor material between the electrodes was between 7 and 30kΩ for each sensor.

Additionally, a pristine SWCNT sensor was fabricated by dropcasting a suspension of SWCNTs in ortho-dichlorobenzene. The suspension was prepared by sonicating SWCNT (0.25mg/mL) in o-dichlorobenzene for 1 minute. The suspension was dropcast until the resistance of each sensor was between 7-10kΩ. Between each successive dropcast of the suspension, the device was vacuum dried until complete solvent removal.

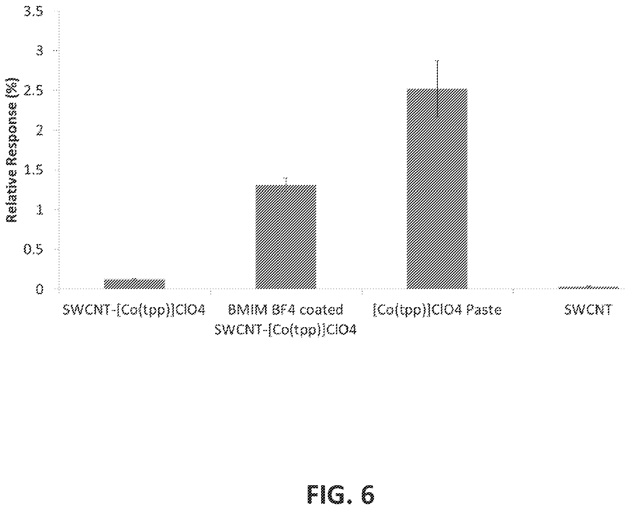

To determine the sensing capability of the devices, the change in conductivity of the sensor was monitored upon exposure to analyte, using the method described in "Materials and Measurements." The sensors were alternatingly exposed to the analyte, 40ppm ethylene in nitrogen, and the carrier gas nitrogen for three cycles **FIG. 6** shows the average sensing response of sensors fabricated using a suspension of SWCNTs and ([Co(tpp)]ClO₄, a suspension of SWCNTs and ([Co(tpp)]ClO₄ coated with BMIM BF₄, a paste of SWCNTs, BMIM BF₄, and ([Co(tpp)]ClO₄, and a suspension of pristine SWCNTs to 40 ppm ethylene.

The average sensing response for the single layered SWCNT-[Co(tpp)]ClO₄ sensors was 0.13%. The BMIM BF₄ coated SWCNT-[Co(tpp)]ClO₄ sensors had an average 1.3% response, thus a ten-fold improvement compared to the uncoated SWCNT-[Co(tpp)]ClO₄ sensor. The [Co(tpp)]ClO₄ paste sensors had an average 2.5% response at 40ppm, with a two-fold improvement compared to the BMIM BF₄ coated sensor and a twenty-fold improvement compared to the uncoated SWCNT-[Co(tpp)]ClO₄ sensor.

As discussed above, this unexpected improvement in the signal is attributed to the improved dispersion of the detector and the carbon nanotubes in the ionic liquid paste. As identified by the applicants, since the ionic liquid does not impede the diffusion of the analyte into the paste, the relative response produced by the paste as a sensor material is significantly stronger than other configurations, such as a simple detector and carbon nanotube combination without the ionic liquid, or a configuration wherein the ionic liquid is coated above the detector and the carbon nanotubes.

Further, it is anticipated that the improved response of the device with the paste as the sensor material will elongate the working life. This is expected since, the sensor has a longer available functioning period before the strength of the response falls below a threshold after which detection of the analyte is not possible.

### Example 5

The following example describes the fabrication and measurement of three types of sensors over the course of four weeks: 1) SWCNT-[Co(tpp)]ClO₄ layer, 2) BMIM BF₄ layered on a SWCNT-[Co(tpp)]ClO₄ layer, and 3) [Co(tpp)]ClO₄ paste.

The substrate was made by depositing a gold electrode pattern, with a 1mm electrode gap, onto a glass slide using a thermal evaporator (Mill Lane Engineering, EV-2000). The electrode pattern was made using a shadow mask and layering 10nm of chromium then 100nm of gold.

The SWCNT-[Co(tpp)]ClO₄ sensors were prepared by dropcasting a SWCNT and [Co(tpp)]ClO₄ suspension. The suspension was prepared by sonicating [Co(tpp)]ClO₄ (10:1 mass ratio of [Co(tpp)]ClO₄ to SWCNT) and 0.25mg/mL SWCNT in o-dichlorobenzene for 7 minutes. The suspension was dropcast until the resistance of each sensor was between 7-10kΩ. Between each successive dropcast of the suspension, the device was vacuum dried until complete solvent removal.

The sensor comprised of a BMIM BF₄ layer on a SWCNT-[Co(tpp)]ClO₄ layer, was prepared by dropcasting the SWCNT-[Co(tpp)]ClO₄ suspension until the resistance of each sensor was between 2 and 5kΩ. Between each dropcast, the device was vacuum dried until solvent was completely removed. A 2 mg/mL BMIM BF₄ solution in methanol was dropcast (1 µL drop) on the SWCNT-[Co(tpp)]ClO₄ layer. The device was vacuum dried to remove the methanol.

The [Co(tpp)]ClO₄ paste sensor was prepared by grinding SWCNT, BMIM BF₄, and [Co(tpp)]ClO₄ for 10min using an agate mortar and pestle. The paste composition was 1wt% of SWCNT in BMIM BF₄ with a 5:1 mass ratio of [Co(tpp)]ClO₄ to SWCNT. The sensors were fabricated by placing the paste between the electrodes using a metal spatula. Additional paste was added until the resistance of the sensor material between the electrodes was between 7 and 30 kΩ for each sensor.

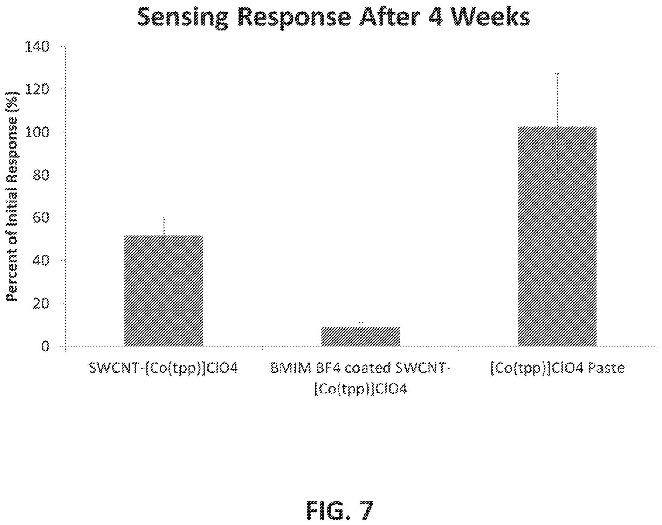

To determine the sensing capability of the devices, the change in conductivity of the sensor was monitored upon exposure to analyte, using the method described in "Materials and Measurements." The sensors were alternatingly exposed to the analyte, 40ppm ethylene in nitrogen, and the carrier gas nitrogen for three cycles. After four weeks, the measurement was repeated. **FIG. 7** shows the percent of initial response of sensors fabricated using a suspension of SWCNTs and ([Co(tpp)]ClO₄, a suspension of SWCNTs and ([Co(tpp)]ClO₄ coated with BMIM BF₄, and a paste of SWCNTs, BMIM BF₄, and ([Co(tpp)]ClO₄ to 40 ppm ethylene four weeks after sensor fabrication.

The average sensing response after this time for the single layered SWCNT-[Co(tpp)]ClO₄ sensors was 51.6% of the initial response. The BMIM BF₄ coated SWCNT-[Co(tpp)]ClO₄ sensors was 8.6 % of the initial response after four weeks. The [Co(tpp)]ClO₄ paste sensors were 102.5% of the initial response at 40ppm.

### Example 6

The following example describes the fabrication and measurement of [Co(tpp)]ClO₄ paste sensors.

The paste was prepared by grinding the SWCNT, BMIM BF₄, and [Co(tpp)]ClO₄ for 20min using an agate mortar and pestle. The composition of the paste was 1wt% SWCNT in BMIM BF₄ and a 5:1 mass ratio of [Co(tpp)]ClO₄ to SWCNT. The substrate was made by depositing a gold electrode pattern, with a 1mm electrode gap, onto a glass slide using a thermal evaporator (Mill Lane Engineering, EV-2000). The electrode pattern was made using a shadow mask and layering 10nm of chromium then 100nm of gold. The sensors were fabricated by placing the paste between the electrodes using a metal spatula. Additional paste was added until the resistance of the sensor material between the electrodes was between 7 and 30kΩ for each sensor.

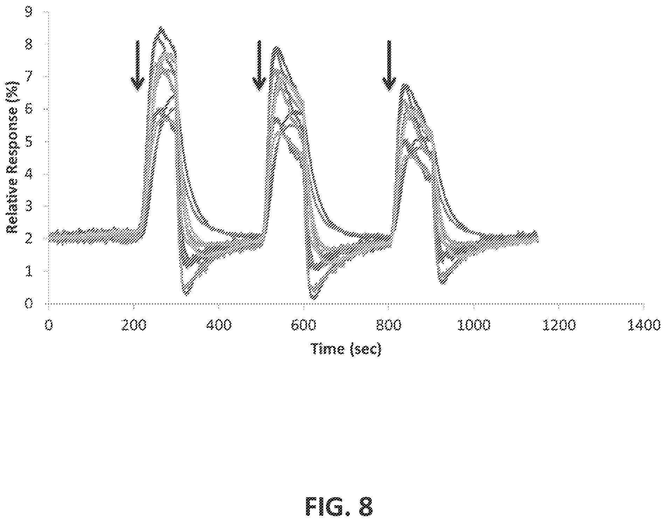

To determine the sensing capability of the devices, the change in conductivity of the sensor was monitored upon exposure to analyte, using the method described in "Materials and Measurements." The sensors were alternatingly exposed to the analyte, 40ppm ethylene in nitrogen, and the carrier gas nitrogen for three cycles. **FIG. 8** shows the sensing response of sensors fabricated using a paste of SWCNTs, BMIM BF₄, and [Co(tpp)]ClO₄ with 1 wt% SWCNT content in BMIM BF₄ and a [Co(tpp)]ClO₄ to SWCNT ratio of 5:1 by mass. Arrows indicate the start of a 100 sec to 40 ppm ethylene. The average sensing response for the sensors was 4.5%.

### Example 7

The following example describes the fabrication and measurement of a [Co(tpp)]ClO₄ paste sensor. The sensor was exposed to ethylene, ethyl acetate, ethanol, hexanes, chloroform, and acetonitrile.

The paste was prepared by grinding SWCNT, BMIM BF₄, and [Co(tpp)]ClO₄ for 20min using an agate mortar and pestle. The composition of the paste was 1wt% SWCNT in BMIM BF₄ and a 5:1 mass ratio of [Co(tpp)]ClO₄ to SWCNT. The substrate was made by depositing a gold electrode pattern, with a 1mm electrode gap, onto a glass slide using a thermal evaporator (Mill Lane Engineering, EV-2000). The electrode pattern was made using a shadow mask and layering 10nm of chromium then 100nm of gold. The sensor was fabricated by placing the paste between the electrodes using a metal spatula. Additional paste was added until the resistance of the sensor material between the electrodes was between 7 and 30kΩ for each sensor.

To determine the sensing capability of the devices, the change in conductivity of the sensor was monitored upon exposure to analyte. The sensors were alternatingly exposed to the analyte, 40ppm ethylene in nitrogen, and the carrier gas nitrogen for three cycles and the response measured using the method described in "Materials and Measurements". The average sensing response to ethylene for the sensors was 4.5%.

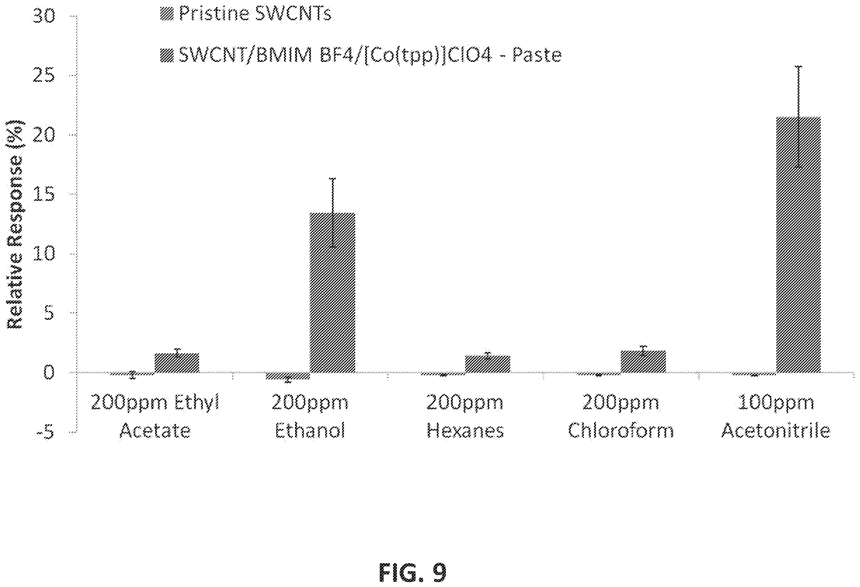

The other analytes gas streams were obtained using a gas generator (Kin-Tek, 491M Base Module). The sensors were alternatingly exposed to the analyte in nitrogen, and the carrier gas nitrogen for three cycles. **FIG. 9** shows the average sensing response of sensors fabricated using a suspension of pristine SWCNTs, and sensors fabricated from a paste of SWCNTs, BMIM BF₄, and [Co(tpp)]ClO₄ to different analytes. The average sensing response to ethyl acetate (200 ppm) was 1.6%. The average response to hexanes (200 ppm) and chloroform (200 ppm) was 1.4% and 1.8%, respectively. The average response to ethanol (200 ppm) was 13.5%. The average response to 100ppm acetonitrile was 21.5%.

### Example 8

The following example describes the fabrication and measurement of a [Co(tpp)]ClO₄ paste sensor, tested at different concentrations of ammonia.

The paste was prepared by grinding SWCNT, BMIM BF4, and [Co(tpp)]ClO₄ using a ball mill. The composition of the paste was 1 wt% SWCNT in BMIM BF₄ and a 5:1 mass ratio of [Co(tpp)]ClO₄ to SWCNT. The electrode pattern was made using a shadow mask and layering 10 nm of chromium then 100 nm of gold. The sensor was fabricated by placing the paste between the electrodes using the screen printing like technique described in "Materials and Methods". The paste was applied in thicknesses of 0.05 mm.

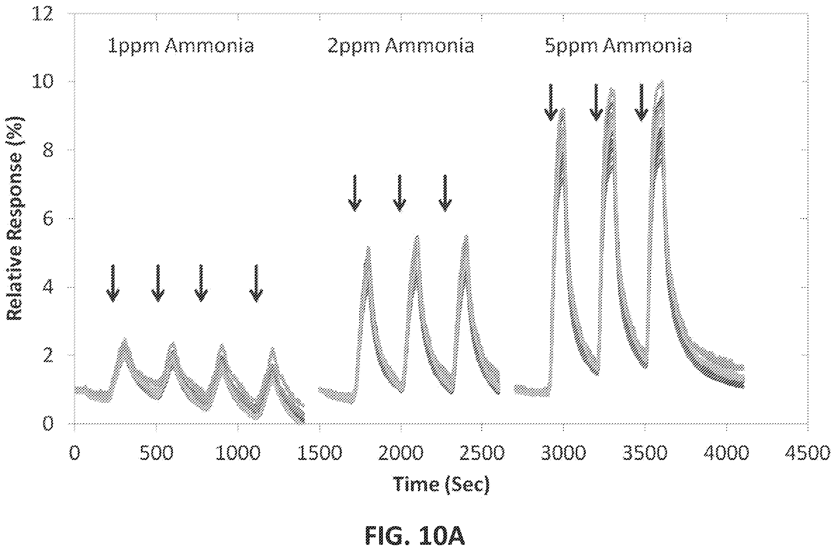

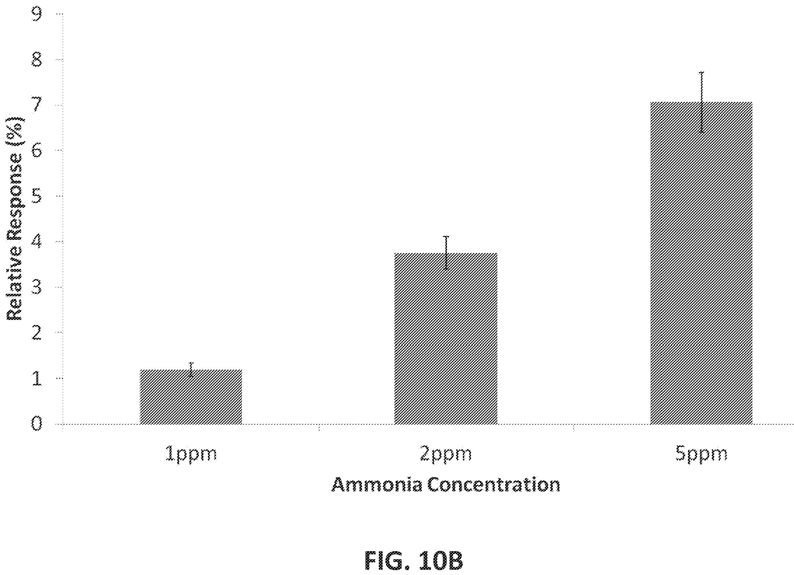

To determine the sensing capability of the devices, the change in conductivity of the sensor was monitored upon exposure to analyte, using the method described in "Materials and Measurements". The sensors were alternatingly exposed to the analyte, 1 ppm, 2 ppm, and 5 ppm ammonia, respectively, in dry nitrogen for 100 sec, and the carrier gas of dry nitrogen for three cycles. **FIG. 10A** shows the sensing response at the various analyte concentrations. The arrows indicate the start of each 100 sec exposure to ammonia. The average sensing response, shown in **FIG. 10B**, is 1.2% ± 0.1% to 1 ppm ammonia, 3.8% ± 0.4 to 2 ppm ammonia, and 7.1% ± .0.7% to 5 ppm ammonia.

### Example 9

The following example describes the fabrication and measurement of a [Co(tpp)]ClO₄ paste sensor, measured at different concentrations of cadaverine.

The paste was prepared by grinding SWCNT, BMIM BF4, and [Co(tpp)]ClO₄ using a ball mill. The composition of the paste was 1 wt% SWCNT in BMIM BF₄ and a 5:1 mass ratio of [Co(tpp)]ClO₄ to SWCNT. The electrode pattern was made using a shadow mask and layering 10 nm of chromium then 100 nm of gold. The sensor was fabricated by placing the paste between the electrodes using the screen printing like technique described in "Materials and Methods". The paste was applied in thicknesses of 0.05 mm.

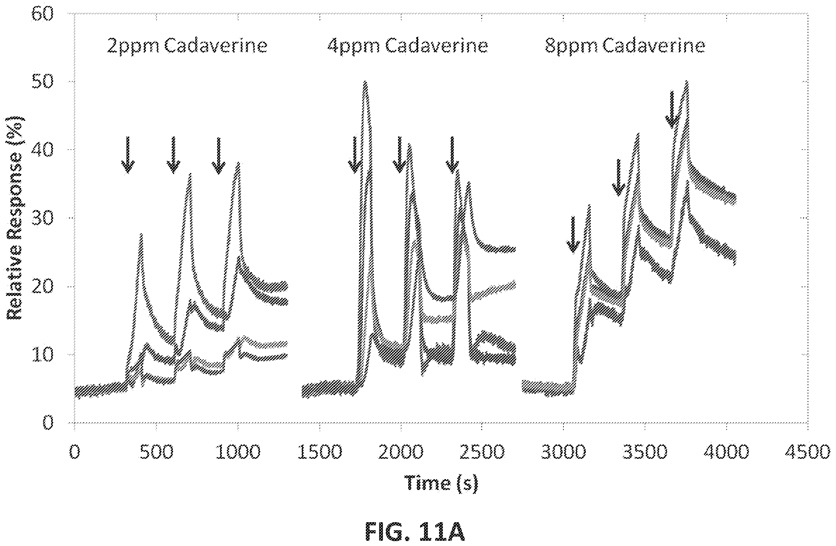

To determine the sensing capability of the devices, the change in conductivity of the sensor was monitored upon exposure to analyte, using the method described in "Materials and Measurements". The sensors were alternatingly exposed to the analyte, 2 ppm, 4 ppm, and 8 ppm cadaverine, respectively, in air with 8500 ppm humidity for 100 sec, and the carrier gas air with 8500 ppm humidity for three cycles. **FIG. 11A** shows the sensing response at the various analyte concentrations. The arrows indicate the start of each 100 sec exposure to cadaverine.

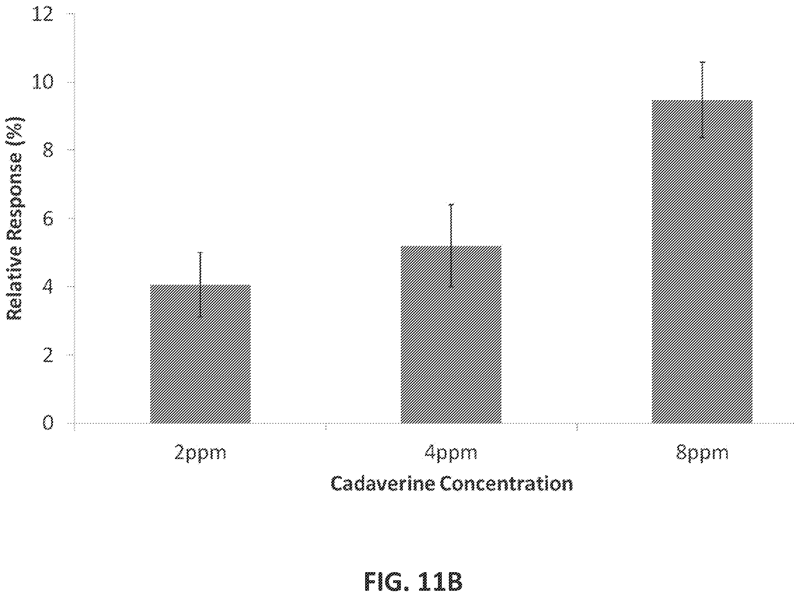

**FIG. 11B** shows the irreversible portion of the sensing response which can be correlated to amine concentration according to the literature (Liu, S. F., Petty, A. R., Sazama, G. T. and Swager, T. M Angew. Chem. Int. Ed., 2015, 54, 6554-6557). Sensing responses of 4.0% ± 1.0%, 5.2% ± 1.2%, and 9.5% ± 1.1% were obtained at 2 ppm, 4 ppm, and 8 ppm cadaverine, respectively.

### Example 10

The following example describes the fabrication and measurement of PdCl₂ paste sensors with different imidazolium-based ionic liquids, tested at 80 ppm ethylene. The ionic liquids used were: 1-ethyl-3-methylimidazolium bis(trifluoromethylsulfonyl)imide (Ethyl TFMS), 1-Butyl-3-methylimidazolium bis(trifluoromethylsulfonyl)imide (Butyl TFMS),1-Hexyl-3-methylimidazolium bis(trifluormethylsulfonyl)imide (Hexyl TFMS).

The pastes were prepared by grinding SWCNT, ionic liquid, and PdCl₂ using a ball mill. The composition of the paste was 1 wt% SWCNT in ionic liquid and a 5:1 mass ratio of PdCl₂ to SWCNT. The ionic liquids tested were BMIM BF₄, 1-Butyl-3-methylimidazolium bis(trifluoromethylsulfonyl)imide (Butyl TFMS), 1-Ethyl-3-methylimidazolium bis(trifluoromethylsulfonyl)imide (Ethyl TFMS), 1-Hexyl-3-methylimidazolium bis(trifluoromethylsulfonyl)imide (Hexyl TFMS). A control sensing material was prepared by drop-casting from a suspension of PdCl₂ and SWCNTs in a 5:1 mass ratio in isopropanol. The suspension was prepared by sonication for 5 minutes.

The electrode pattern was made using a shadow mask and layering 10 nm of chromium then 100 nm of gold.. The sensor was fabricated by placing the paste between the electrodes using the screen printing like technique described in "Materials and Methods". The paste was applied in thicknesses of 0.05 mm.The control sensor was fabricated by drop-casting the control sensing material between the electrodes. Between each successive dropcast the device was allowed to dry in air. The suspension was dropcast until the resistance of each sensor was between 0.95 and 1.5 kΩ.

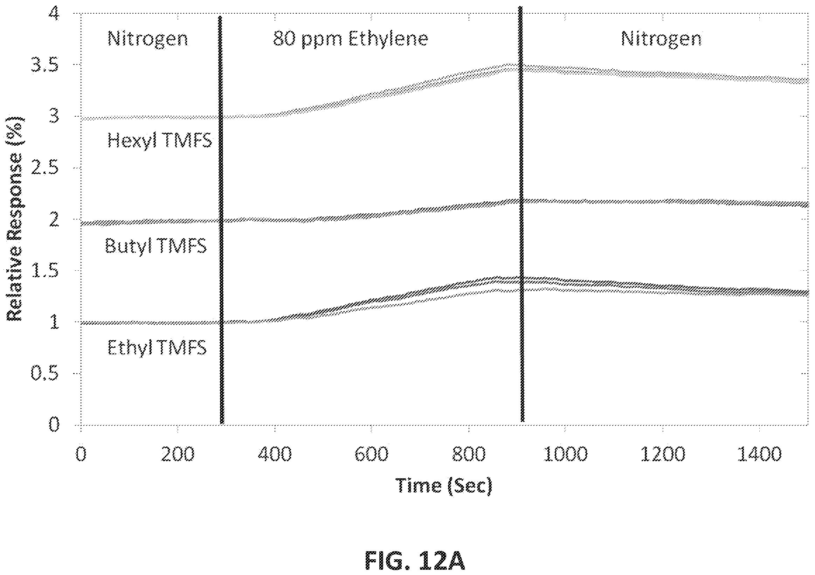

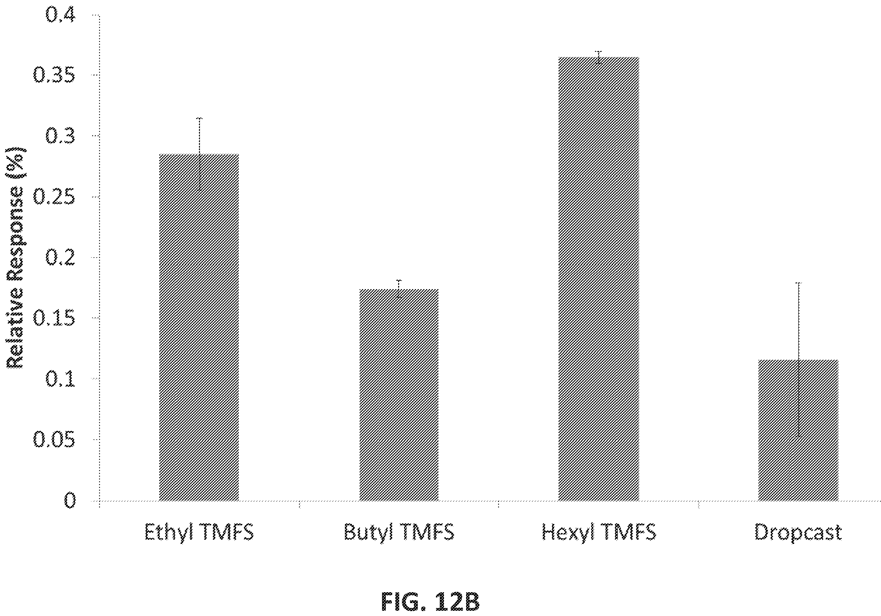

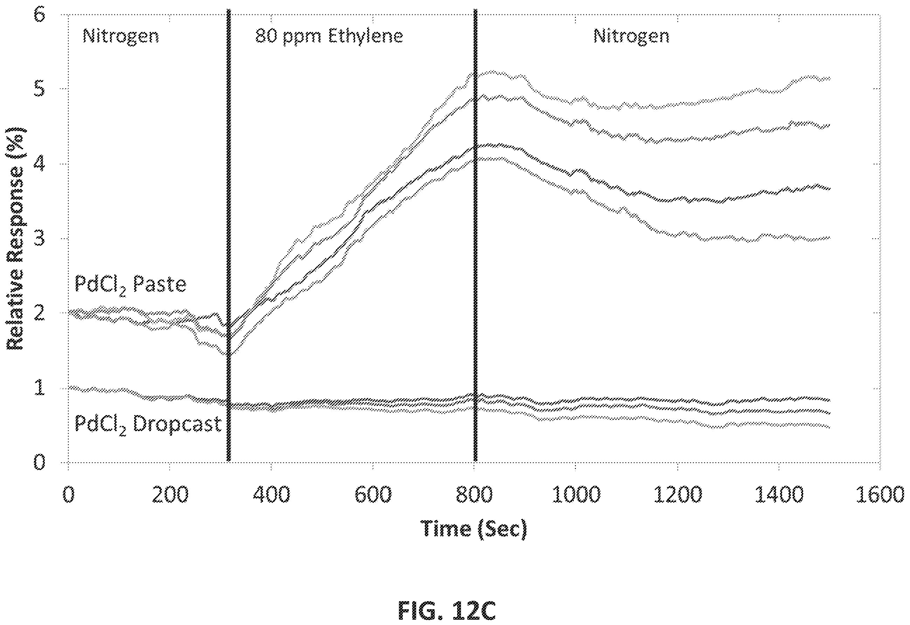

To determine the sensing capability of the devices, the change in conductivity of the sensor was monitored upon exposure to analyte, using the method described in "Materials and Measurements". The sensors were exposed to the analyte, 80 ppm ethylene in nitrogen with 10,000 ppm humidity for 500 sec, and the carrier gas of nitrogen with 10,000 ppm humidity for one cycle. **FIG. 12A** shows the sensing response of pastes with the various ionic liquids. The lines indicate the start and end of the 500 sec exposure to ethylene. The relative response, shown in **FIG. 12B****,** for Butyl TFMS is 0.17% ± 0.01%. The response for Ethyl TFMS is 0.28% ± 0.03%. The response for Hexyl TFMS is 0.36% ± 0.005%. This suggests that the polarity and viscosity of the ionic liquid which is influenced by its alkyl side-chain has an effect on the sensing performance. The response for the dropcast PdCl₂ sensor is 0.11% ± 0.06%. **FIG. 12C** shows the sensing response for the control sensor of dropcast PdCl₂ and the BMIM BF₄ paste sensor. The lower sensing response and higher error of the dropcast control sensor indicates that the use of sensor material pastes is superior to alternative methods such as drocasting a mixture of SWCNTs and the detector.

### Example 11

Similar to Example 10, the following example describes the fabrication and measurement of PdCl₂ paste sensors with 1-Butyl-3-methylimizaolium hexafluorophosphate (Butyl HFP), tested at 80 ppm ethylene.

The pastes were prepared by grinding SWCNT, Butyl HFP, and PdCl₂ using a ball mill. The composition of the paste was 1 wt% SWCNT in Butyl HFP and a 5:1 mass ratio of PdCl₂ to SWCNT. The electrode pattern was made using a shadow mask and layering 10 nm of chromium then 100 nm of gold. The sensor was fabricated by placing the paste between the electrodes using the screen printing like technique described in "Materials and Methods". The paste was applied in thicknesses of 0.05 mm.

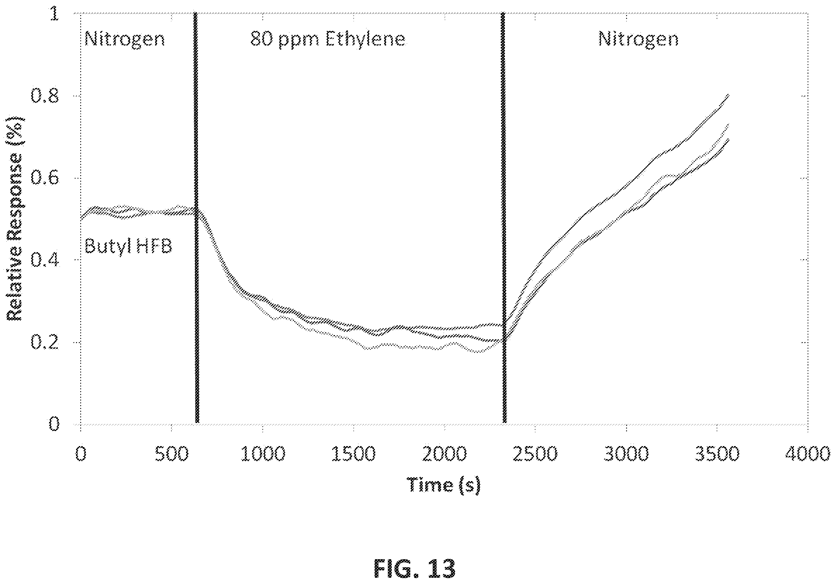

To determine the sensing capability of the devices, the change in conductivity of the sensor was monitored upon exposure to analyte, using the method described in "Materials and Measurements". The sensors were alternatingly exposed to the analyte, 80 ppm ethylene in nitrogen with 10,000 ppm humidity for 800 sec, and the carrier gas of nitrogen with 10,000 ppm humidity for one cycle. **FIG. 13** shows the sensing response of the Butyl HFP pastes. The lines indicate the start and end of the 800 sec exposure to ethylene. The relative response for Butyl HFP was -0.226% ± 0.01%.

### Example 12

Similar to Example 10, the following example describes the fabrication and measurement of [Co(tpp)]ClO₄ paste sensors with different imidazolium-based ionic liquids, tested at 80 ppm ethylene. The ionic liquids used were: 1-ethyl-3-methylimidazolium bis(trifluoromethylsulfonyl)imide (Ethyl TFMS), 1-Butyl-3-methylimidazolium bis(trifluoromethylsulfonyl)imide (Butyl TFMS), 1 -Hexyl-3-methylimidazolium bis(trifluormethylsulfonyl)imide (Hexyl TFMS).

The pastes were prepared by grinding SWCNT, ionic liquid, and [Co(tpp)]ClO₄ using a ball mill. The composition of the paste was 1 wt% SWCNT in ionic liquid and a 5:1 mass ratio of [Co(tpp)]ClO₄ to SWCNT. The ionic liquids tested were Butyl TFMS, Ethyl TFMS, and Hexyl TFMS. The electrode pattern was made using a shadow mask and layering 10 nm of chromium then 100 nm of gold. The sensor was fabricated by placing the paste between the electrodes using The sensor was fabricated by placing the paste between the electrodes using the screen printing like technique described in "Materials and Methods". The paste was applied in thicknesses of 0.05 mm.

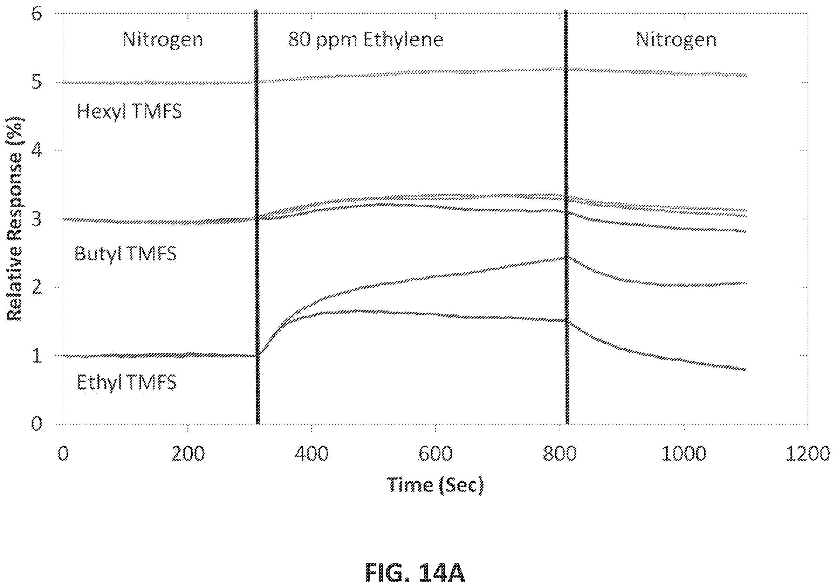

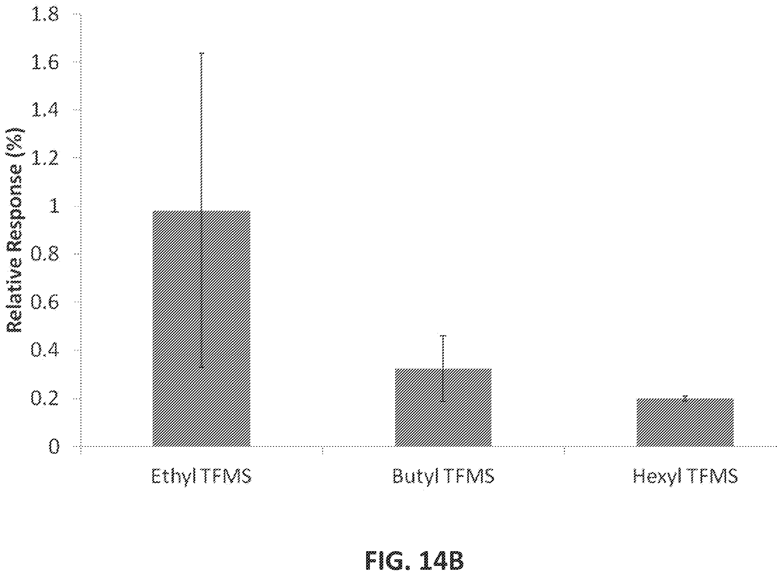

To determine the sensing capability of the devices, the change in conductivity of the sensor was monitored upon exposure to analyte, using the method described in "Materials and Measurements". The sensors were alternatingly exposed to the analyte, 80 ppm ethylene in dry nitrogen for 500 sec, and the carrier gas of dry nitrogen for one cycle. **FIG. 14A** shows the sensing response of the various ionic liquids. The lines indicate the start and end of the 500 sec exposure to ethylene. The relative response for Butyl TFMS was 0.3% ± 0.1%. The response for Ethyl TFMS was 1.0% ± 0.7%. The response for Hexyl TFMS was 0.20% ± 0.01%. Relative responses of the three ionic liquid pastes are displayed in **FIG. 14B****.**

### Example 13

The following example describes the fabrication and measurement of a [Co(tpp)]ClO₄ paste sensor, tested with ethylene at different points during the fabrication process.

The paste was prepared by grinding SWCNT, BMIM BF4, and [Co(tpp)]ClO₄ using an agate mortar and pestle. The composition of the paste was 1 wt% SWCNT in BMIM BF₄ and a 5:1 mass ratio of [Co(tpp)]ClO₄ to SWCNT. The electrode pattern was made using a shadow mask and layering 10nm of chromium then 100nm of gold. The sensor was fabricated by placing the paste between the electrodes using a metal spatula. The paste was applied after 1, 4, 7, 10, 13, 17, and 20 min of grinding. Paste was added until the resistance of the sensor material between the electrodes was between 7 and 30kΩ for each sensor.

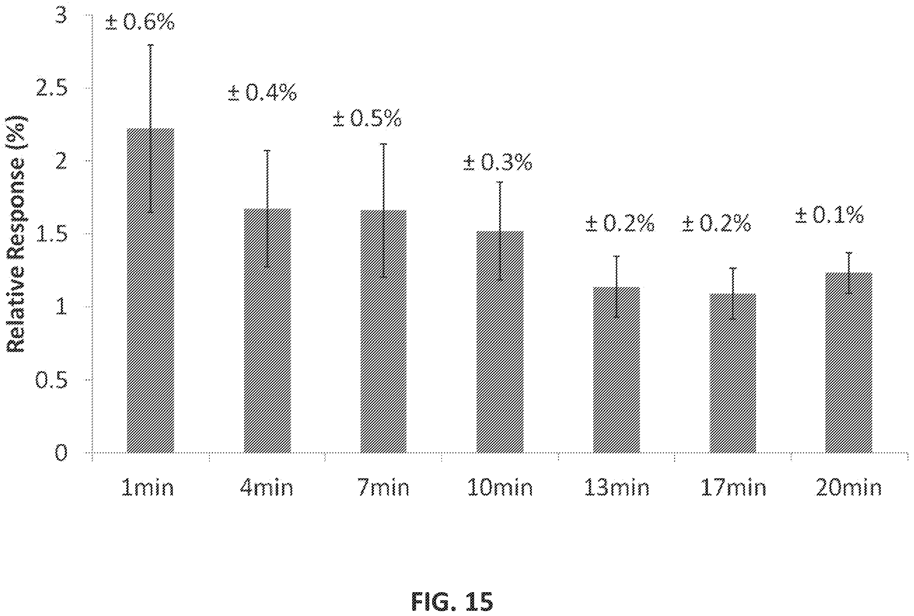

To determine the sensing capability of the devices, the change in conductivity of the sensor was monitored upon exposure to analyte, using the method described in "Materials and Measurements". The sensors were alternatingly exposed to the analyte, 40ppm ethylene in nitrogen for 100sec, and the carrier gas nitrogen for three cycles. FIG. 15 shows the average sensing response at the various grinding times, with the standard deviation shown for each time point. Although the sensing response decreases with more mixing, standard deviation of the response also decreases with additional grinding time.

### Example 14

The following example describes the fabrication and measurement of a PdCl₂ paste sensor, tested with ethylene at different points during the fabrication process.

The paste was prepared by grinding SWCNT, BMIM BF4, and PdCl₂ using a ball mill. The composition of the paste was 0.25 wt% SWCNT in BMIM BF₄ and a 5:1 mass ratio of PdCl₂ to SWCNT. The electrode pattern was made using a shadow mask and layering 10nm of chromium then 100nm of gold. The sensor was fabricated by placing the paste between the electrodes using a metal spatula. The paste was applied after 1, 3, 5, 7, and 10 min of grinding. Sensor material was added until the resistance of the material between the electrodes was between 7 and 20kΩ for each sensor.

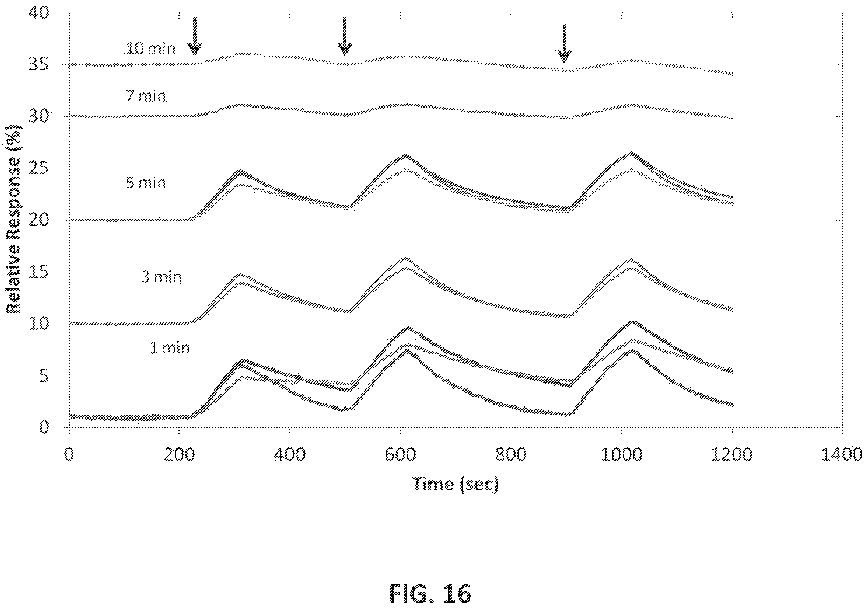

To determine the sensing capability of the devices, the change in conductivity of the sensor was monitored upon exposure to analyte, using the method described in "Materials and Measurements". The sensors were alternatingly exposed to the analyte, 40ppm ethylene in humidified nitrogen (10,000ppm water) for 100sec, and the carrier gas of humidified nitrogen for three cycles. **FIG. 16** shows the sensing response at the various grinding times. The arrows indicate the start of each 100 sec exposure to 40 ppm ethylene. The sensing response decreases with additional grinding: an average of 5.2% after 1 min, 4.7% after 3 min, 4.6% after 5 min, 1.1% after 7 min, 0.9% after 10 min of grinding.

### Example 15

The following example describes the fabrication and measurement of a PdCl₂ paste sensor, applied at differing thickness of sensing material.

The paste was prepared by grinding SWCNT, BMIM BF₄, and PdCl₂ using a ball mill. The composition of the paste was 1 wt% SWCNT in BMIM BF₄ and a 5:1 mass ratio of PdCl₂ to SWCNT. The electrode pattern was made using a shadow mask and layering 10nm of chromium then 100nm of gold. The sensor was fabricated by placing the paste between the electrodes using the screen printing like technique described in "Materials and Methods". The paste was applied in thicknesses of 0.05, 0.10, and 0.15 mm, by varying the thickness of the screen printing mask.

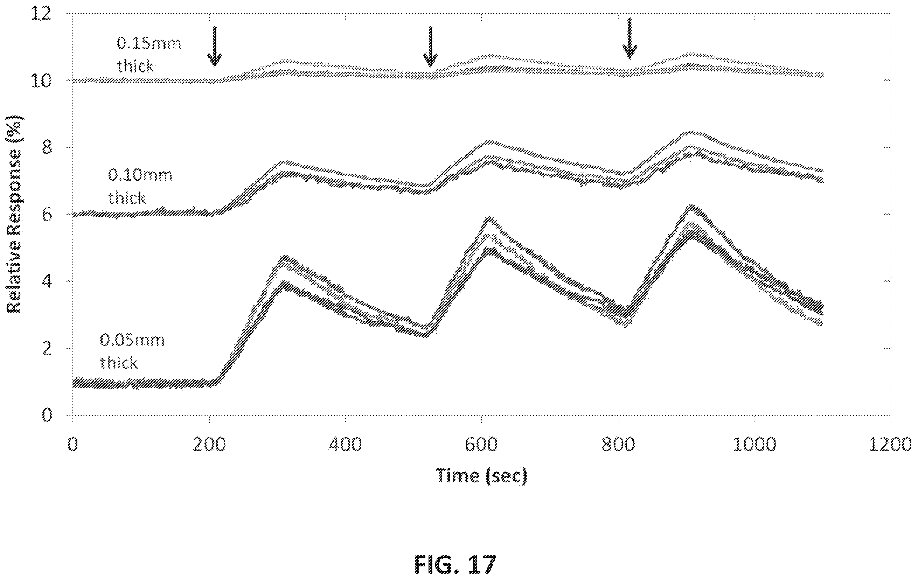

To determine the sensing capability of the devices, the change in conductivity of the sensor was monitored upon exposure to analyte, using the method described in "Materials and Measurements". The sensors were alternatingly exposed to the analyte, 40ppm ethylene in humidified nitrogen (10,000ppm water) for 100sec, and the carrier gas of humidified nitrogen for three cycles. **FIG. 17** shows the sensing response at the different thicknesses. The arrows indicate the start of each 100 sec exposure to 40 ppm ethylene. The sensing response decreases as the thickness increases, from an average of 3.0% with a 0.05 mm thickness, to 1.6% with a 0.10 mm thickness, to 0.3% with a 0.15 mm thickness.

## Claims

1. A sensor material comprising:
a plurality of conductive carbon nanotubes;
a detector capable of interaction with an analyte of interest, the detector being an organic, an organometallic or an inorganic species, and the detector comprising a functional group capable of binding an analyte of interest in a solution, vapor phase or solid phase; and
an ionic liquid,
wherein the plurality of carbon nanotubes, the detector and the ionic liquid are combined to form a paste;
the ionic liquid is selected to facilitate analyte interaction with the paste resulting in a change of the conductivity of the paste; and
the carbon nanotubes are mixed with the detector in a ratio ranging from 3:1 to 1:10 by weight.

2. The sensor material of Claim 1, wherein the carbon nanotubes are mixed with the detector in a ratio ranging from 1:1 to 1:10 by weight.

3. The sensor material of Claim 1 or Claim 2, wherein the ionic liquid:
a) comprises cations selected from the group consisting of imidazolium cations, pyridinium cations, pyrrolidinium cations, phosphonium cations, and combinations thereof; or
b) comprises an anion selected from the group consisting of bis(trifluoromethanesulfonyl)imide (TFSI-) anions, bis(fluorosulfonyl)imide (FSI-) anions, halide anions, nitrate anions, tetrafluoroborate anions, hexafluorophosphate anions, bistriflimide anions, triflate anions, tosylate anions and combinations thereof; or
c) comprises non-halogenated organic anions selected from a group consisting of formate, alkylsulfate, alkylphosphate, glycolate and combinations thereof; or
d) is 1-butyl-3- methylimidazolium tetrafluoroborate, 1-butyl-3-methylimidazolium hexafluorophosphate, 1-ethyl-3-methylimidazolium bis(trifluoromethylsulfonyl)imide, 1-Butyl-3-methylimidazolium bis(trifluoromethylsulfonyl)imide, or 1-hexyl-3-methylimidazolium bis(trifluormethylsulfonyl)imide.

4. The sensor material according to any preceding claim, wherein the detector:
a) is covalently bonded to the carbon nanotubes; or
b) is non-covalently bonded to the carbon nanotubes; or
c) is constricted inside the carbon nanotubes; or
d) is a small molecule, a polymer, or a biological species; or
f) is electron-rich or electron-poor moiety; wherein interaction between an analyte of interest and the detector comprises an electrostatic interaction; or
g) comprises a metal or metal-containing compound; or
h) is PdCl₂ or 5,10,15,20-tetraphenylporphyrinatocoblat(III) perchlorate ([Co(tpp)]ClO₄), 3,6-Di-2-pyridyl-1,2,4,5-tetrazine.

5. The sensor material of Claim 1, wherein the functional group is selected from the group consisting of a thiol, an aldehyde, an ester, a carboxylic acid, a hydroxyl group or a combination thereof.

6. The sensor material of Claim 4g, wherein:
a) interaction between an analyte of interest and the detector comprises binding to the metal or metal-containing compound;
b) the metal containing compound is selected from a group consisting of titanium salts, silver salts, platinum salts, gold salts, aluminum salts, nickel salts, palladium salts, and copper salts; or
c) the metal containing compound comprises a copper salt; or
d) the metal containing compound comprises a palladium salt.

7. The sensor material of Claim 4d, wherein the biological species comprises a peptide, protein, DNA, RNA or PNA.

8. The sensor material of any one of Claims 1-4:
a) wherein 0.1 to 20 weight% of the carbonaceous nanomaterial particles are mixed with the ionic liquid; or
b) wherein 0.25 to 10 weight% of the carbon nanotubes are mixed with the ionic liquid; or
c) further comprising viscosity modifier additives.

9. The sensor material of Claim 8c, wherein the viscosity modifier additive is selected from the group consisting of low molecular weight solvents, high molecular weight solvents, plasticizers, ethylene glycol, tetraethylene glycol, thinners and mineral oils.

10. A device comprising:
a first electrode and a second electrode; and
a sensor material disposed in electrical contact with the first and second electrode,
wherein the sensor material comprises the sensor material as defined in any preceding claim.

11. The device of Claim 10, further including an electrical circuit in connection with an ammeter or voltmeter to detect the change in conductivity of the paste forming the sensor material.

12. The device of Claim 10, wherein the first and second electrodes are:
a) located on a rigid substrate; or
b) located on a flexible substrate; or
c) are part of a complex circuit.

13. The device of Claim 10 wherein:
a) the rigid substrate of Claim 12a is selected from glass, polymeric material and printed circuit board; or
b) the flexible substrate of Claim 12b is paper or a polymeric material; or
c) the complex circuit of Claim 12c is a Near Field Communication (NFC) chip or a radio-frequency identification (RFID) chip.

14. A method of detecting an analyte comprising:
providing a sensing device according to any of Claims 10-13;
exposing the sensor material to an environment, wherein a change in the conductivity of the sensor material indicates the presence of the analyte; and
detecting said change in conductivity of the sensor material.

15. The method of Claim 14, further comprising:
a) transmitting the detected changes in conductivity wirelessly to another device for analysis and storage;
b) detecting the analyte through a wireless radio frequency communication; and
c) detecting an output from a radio frequency identification tag including the sensor.

16. The method of Claim 14, wherein the analyte is:
a) a vapor; or
b) selected from the group consisting of a thiol, an ester, an aldehyde, an alcohol, an ether, an alkene, an alkyne, a ketone, an acid, a base, and a combination thereof; or
c) a mold; or
d) ethylene, a nitrogen-containing gas or an amine; or
e) putrescine or cadaverine; or
f) in the range of 0 to 10%, 0 to 5%, 0 to 1%, 0 to 1000 ppm, 0 to 100 ppm, 0 to 80 ppm, 0 to 50 ppm, 0 to 10 ppm, 0 to 5ppm, 0 to 1 ppm, 0 to 0.5 ppm, 0 to 100 ppb, 0 to 50 ppb, or 0 to 10 ppb.

17. The method of Claim 14, wherein the sensor material further undergoes:
a) a volumetric change upon interaction with the analyte; and
detecting the volumetric change and deriving information regarding the analyte from said volumetric change; or
b) a color change upon interaction with the analyte; and
detecting the color change and deriving information regarding the analyte from said color change.

18. The method of Claim 14, wherein the analyte interacts with the detector:
a) to form a Van der Waals interaction, a covalent bond, an ionic bond, a hydrogen bond or a dative bond; or
b) via a binding event between pairs of biological molecules, wherein the biological molecules are proteins, nucleic acids, glycoproteins, carbohydrates or hormones.

## Patentansprüche

1. Sensormaterial, enthaltend:
eine Vielzahl von leitenden Kohlenstoff-Nanoröhren,
einen Detektor, der mit einem interessierenden Analyten interagieren kann, wobei der Detektor eine organische, organometallische oder anorganische Spezies ist und der Detektor eine funktionelle Gruppe enthält, die in der Lage ist, einen interessierenden Analyten in einer Lösung, Dampfphase oder festen Phase zu binden, und
eine ionische Flüssigkeit,
worin die Vielzahl von Kohlenstoff-Nanoröhren, der Detektor und die ionische Flüssigkeit zur Bildung einer Paste kombiniert sind,
die ionische Flüssigkeit ausgewählt ist, zum Erleichtern der Analytinteraktion mit der Paste, was zu einer Änderung der Leitfähigkeit der Paste führt, und
die Kohlenstoff-Nanoröhren mit dem Detektor in einem Gewichtsverhältnis im Bereich von 3:1 bis 1:10 gemischt sind.

2. Sensormaterial gemäß Anspruch 1, worin die Kohlenstoff-Nanoröhren mit dem Detektor in einem Gewichtsbereich von 1:1 bis 1:10 gemischt sind.

3. **Sensormaterial** gemäß Anspruch 1 oder 2, worin die ionische Flüssigkeit:
a) Kationen enthält, ausgewählt aus der Gruppe, bestehend aus Imidazoliumkationen, Pyridiniumkationen, Pyrrolidiniuimkationen, Phosphoniumkationen und Kombinationen davon oder
b) ein Anion enthält, ausgewählt aus der Gruppe, bestehend aus Bis(trifluormethansulfonyl)imid (TFSI-)-Anionen, Bis(fluorsulfonyl)imid (FSI-)-Anionen, Halogenidanionen, Nitratanionen, Tetrafluorboratanionen, Hexafluorphosphatanionen, Bistriflimidanionen, Triflatanionen, Tosylatanionen und Kombinationen davon oder
c) nicht-halogenierte organische Anionen enthält, ausgewählt aus der Gruppe, bestehend aus Formiat, Alkylsulfat, Alkylphosphat, Glycolat und Kombinationen davon oder
d) 1-Butyl-3-methylimidazoliumtetrafluorborat, 1-Butyl-3-methylimidazoliumhexafluorphosphat, 1-Ethyl-3-methylimidazolium-bis(trifluormethylsulfonyl)imid, 1-Butyl-3-methylimidazolium-bis(trifluormethylsulfonyl)imid oder 1-Hexyl-3-methylimidazolium-bis(trifluormethylsulfonyl)imid ist.

4. Sensormaterial gemäß einem der vorhergehenden Ansprüche, worin der Detektor:
a) kovalent an die Kohlenstoff-Nanoröhren gebunden ist oder
b) nicht-kovalent an die Kohlenstoff-Nanoröhren gebunden ist oder
c) im Inneren der Kohlenstoff-Nanoröhren eingeengt ist oder
d) ein kleines Molekül, Polymer oder eine biologische Spezies ist oder
f) ein elektronenreicher oder elektronenarmer Anteil ist, worin die Interaktion zwischen einem interessierenden Analyten und dem Detektor eine elektrostatische Interaktion enthält, oder
g) eine Metall- oder eine Metall-haltige Verbindung enthält oder
h) PdCl₂ oder 5,10,15,20-Tetraphenylporphyrinatocobalt(III)perchlorat ([Co(tpp)]ClO₄), 3,6-Di-2-pyridyl-1,2,4,5-tetrazin ist.

5. Sensormaterial gemäß Anspruch 1, worin die funktionelle Gruppe ausgewählt ist aus der Gruppe, bestehend aus einem Thiol, Aldehyd, Ester, Carbonsäure, Hydroxylgruppe oder einer Kombination davon.

6. Sensormaterial gemäß Anspruch 4g, worin:
a) eine Interaktion zwischen einem interessierenden Analyten und dem Detektor das Binden an die Metall-oder Metall-haltige Verbindung enthält,
b) die Metall-haltige Verbindung ausgewählt ist aus einer Gruppe, bestehend aus Titansalzen, Silbersalzen, Platinsalzen, Goldsalzen, Aluminiumsalzen, Nickelsalzen, Palladiumsalzen und Kupfersalzen oder
c) die Metall-haltige Verbindung ein Kupfersalz enthält oder
d) die Metall-haltige Verbindung ein Palladiumsalz enthält.

7. Sensormaterial gemäß Anspruch 4d, worin die biologische Spezies ein Peptid, Protein, DNA, RNA oder PNA enthält.

8. Sensormaterial gemäß einem der Ansprüche 1 bis 4,
a) worin 0,1 bis 20 Gew.-% der Kohlenstoff-haltigen Nanomaterialteilchen mit der ionischen Flüssigkeit gemischt sind oder
b) worin 0,25 bis 10 Gew.-% der Kohlenstoff-Nanoröhren mit der ionischen Flüssigkeit gemischt sind oder
c) weiterhin enthaltend Viskositäts-modifizierende Additive.

9. Sensormaterial gemäß Anspruch 8c, worin das Viskositäts-modifizierende Additiv ausgewählt ist aus der Gruppe, bestehend aus niedermolekularen Lösungsmitteln, hochmolekularen Lösungsmitteln, Plastifizierern, Ethylenglykol, Tetraethylenglykol, Verdünnern und Mineralölen.

10. Vorrichtung, enthaltend:
eine erste Elektrode und eine zweite Elektrode und
ein Sensormaterial, das in elektrischem Kontakt mit der ersten und der zweiten Elektrode angeordnet ist, worin das Sensormaterial das Sensormaterial wie in einem der vorhergehenden Ansprüche enthält.

11. Vorrichtung gemäß Anspruch 10, weiterhin enthaltend einen elektrischen Schaltkreis im Zusammenhangmit einem Amperemeter oder Voltmeter, zum Ermitteln der Änderung der Leitfähigkeit der Paste, die das Sensormaterial bildet.

12. Vorrichtung gemäß Anspruch 10, worin die erste und die zweite Elektrode:
a) auf einem festen Substrat lokalisiert sind oder
b) auf einem flexiblen Substrat lokalisiert sind oder
c) Teil eines komplexen Schaltkreises sind.

13. Vorrichtung gemäß Anspruch 10, worin
a) das feste Substrat gemäß Anspruch 12a ausgewählt ist aus Glas, polymerem Material und gedruckter Schaltplatte oder
b) das flexible Substrat gemäß Anspruch 12b Papier oder ein polymeres Substrat ist oder
c) der komplexe Schaltkreis gemäß Anspruch 12c ein Near-Field-Communication (NFC)-Chip oder ein Radiofrequenz-Identifizierungs (RFD)-Chip ist.

14. Verfahren zum Ermitteln eines Analyten, enthaltend:
Vorsehen einer Sensorvorrichtung gemäß einem der Ansprüche 10 bis 13,
Aussetzen des Sensormaterials gegenüber einer Umgebung, worin eine Änderung der Leitfähigkeit des Sensormaterials das Vorhandensein des Analyten anzeigt und
Ermitteln der Änderung der Leitfähigkeit des Sensormaterials.

15. Verfahren gemäß Anspruch 14, weiterhin enthaltend:
a) drahtloses Transmittieren der ermittelten Änderungen der Leitfähigkeit zu einer anderen Vorrichtung für die Analyse und Speicherung,
b) Ermitteln des Analyten durch eine drahtlose Radiofrequenzkommunikation und
c) Ermitteln eines Ausstoßes von einem Radiofrequenzidentifizierungs-Tag, der den Sensor enthält.

16. Verfahren gemäß Anspruch 14, worin der Analyt:
a) ein Dampf ist,
b) ausgewählt ist aus der Gruppe, bestehend aus einem Thiol, Ester, Aldehyd, Alkohol, Ether, Alken, Alkin, Keton, einer Säure, Base oder einer Kombination davon oder
c) eine Form oder
d) Ethylen, ein Stickstoff-haltiges Gas oder ein Amin oder
e) dem Putrescin oder Cadaverin ist oder
f) im Bereich von 0 bis 10 %, 0 bis 5 %, 0 bis 1 %, 0 bis 1000 ppm, 0 bis 100 ppm, 0 bis 80 ppm, 0 bis 50 ppm, 0 bis 10 ppm, 0 bis 5 ppm, 0 bis 1 ppm, 0 bis 0,5 ppm, 0 bis 100 ppb, 0 bis 50 ppb oder 0 bis 10 ppb ist.

17. Verfahren gemäß Anspruch 14, worin das Sensormaterial weiterhin:
a) eine volumetrische Änderung bei Interaktion mit dem Analyten und Ermitteln der volumetrischen Änderung und Ableiten von Information in Bezug auf den Analyten von der volumetrischen Änderung oder
b) eine Farbänderung bei Interaktion mit dem Analyten und Ermitteln der Farbänderung und Ableiten von Information in Bezug auf den Analyten von der Farbänderung eingeht.

18. Verfahren gemäß Anspruch 14, worin der Analyt mit dem Detektor interagiert:
a) zur Bildung einer Van-der-Waals-Interaktion, einer kovalenten Bindung, ionischen Bindung, Wasserstoffbindung oder koordinierten Bindung, oder
b) über ein Bindungsevent zwischen Paaren von biologischen Molekülen, worin die biologischen Moleküle Proteine, Nukleinsäuren, Glykoproteine, Kohlenhydrate oder Hormone sind.

## Revendications

1. Matériau capteur comprenant :
une pluralité de nanotubes de carbone conducteurs ;
un détecteur capable d'interagir avec un analyte d'intérêt, le détecteur étant une espèce organique, organométallique ou inorganique, et le détecteur comprenant un groupe fonctionnel capable de lier un analyte d'intérêt dans une solution, en phase vapeur ou en phase solide ; et
un liquide ionique,
dans lequel la pluralité de nanotubes de carbone, le détecteur et le liquide ionique sont combinés pour former une pâte ;
le liquide ionique est sélectionné pour faciliter une interaction d'analyte avec la pâte engendrant un changement de la conductivité de la pâte ; et
les nanotubes de carbone sont mélangés avec le détecteur à un rapport dans une plage de 3:1 à 1:10 en poids.

2. Matériau capteur selon la revendication 1, dans lequel les nanotubes de carbone sont mélangés avec le détecteur à un rapport dans une plage de 1:1 à 1:10 en poids.

3. Matériau capteur selon la revendication 1 ou la revendication 2, dans lequel le liquide ionique :
a) comprend des cations sélectionnés dans le groupe consistant en cations imidazolium, cations pyridinium, cations pyrrolidinium, cations phosphonium, et des combinaisons de ceux-ci ; ou
b) comprend un anion sélectionné dans le groupe consistant en anions bis(trifluorométhanesulfonyl)imide (TFSI-), anions bis(fluosulfonyl)imide (FSI-), anions d'halogénure, anions de nitrate, anions de tétrafluoroborate, anions d'hexafluorophosphate, anions de bistriflimide, anions de triflate, anions de tosylate et des combinaisons de ceux-ci ; ou
c) comprend des anions organiques non halogénés sélectionnés dans un groupe consistant en formate, alkylsulfate, alkylphosphate, glycolate et des combinaisons de ceux-ci ; ou
d) est 1-butyl-3-méthylimidazolium tétrafluoroborate, 1-butyl-3-méthylimidazolium hexafluorophosphate, 1-éthyl-3-méthylimidazolium bis(trifluorométhylsulfonyl)imide, 1-Buthyl-3-méthylimidazolium bis(trifluorométhylsulfonyl)imide, ou 1-hexyl-3-méthylimidazolium bis(trifluorométhylsulfonyl)imide.

4. Matériau capteur selon une quelconque revendication précédente, dans lequel le détecteur :
a) est lié de manière covalente aux nanotubes de carbone ; ou
b) est lié de manière non covalente aux nanotubes de carbone ; ou
c) est contraint à l'intérieur des nanotubes de carbone ; ou
d) est une petite molécule, un polymère ou une espèce biologique ; ou
f) est une fraction riche en électrons ou pauvre en électrons ; dans lequel une interaction entre un analyte d'intérêt et le détecteur comprend une interaction électrostatique ; ou
g) comprend un métal ou un composé contenant un métal ; ou
h) est PdCl₂ ou 5,10,15,20-tétraphénylporphyrinatocobalt(III) perchlorate ([Co(tpp)]ClO₄), 3,6-Di-2-pyridyl-1,2,4,5-tétrazine.

5. Matériau capteur selon la revendication 1, dans lequel le groupe fonctionnel est sélectionné parmi le groupe consistant en un thiol, un aldéhyde, un ester, un acide carboxylique, un groupe hydroxyle ou une combinaison de ceux-ci.

6. Matériau capteur selon la revendication 4g, dans lequel :
a) une interaction entre un analyte d'intérêt et le détecteur comprend une liaison au métal ou au composé contenant un métal ;
b) le composé contenant un métal est sélectionné dans un groupe consistant en sels de titane, sels d'argent, sels de platine, sels d'or, sels d'aluminium, sels de nickel, sels de palladium et sels de cuivre ; ou
c) le composé contenant un métal comprend un sel de cuivre ; ou
d) le composé contenant un métal comprend un sel de palladium.

7. Matériau capteur selon la revendication 4d, dans lequel l'espèce biologique comprend un peptide, une protéine, ADN, ARN ou ANP.

8. Matériau capteur selon l'une quelconque des revendications 1 à 4 :
a) dans lequel de 0,1 à 20 % en poids des particules de nanomatériau carboné sont mélangées avec le liquide ionique ; ou
b) dans lequel de 0,25 à 10 % en poids des nanotubes de carbone sont mélangés avec le liquide ionique ; ou
c) comprenant en outre des additifs modificateurs de viscosité.

9. Matériau capteur selon la revendication 8c, dans lequel l'additif modificateur de viscosité est sélectionné dans le groupe consistant en solvants à faible poids moléculaire, solvants à fort poids moléculaire, plastifiants, éthylène glycol, tétraéthylène glycol, diluants et huiles minérales.

10. Dispositif comprenant :
une première électrode et une seconde électrode ; et
un matériau capteur disposé en contact électrique avec les première et seconde électrodes,
dans lequel le matériau capteur comprend le matériau capteur selon une quelconque revendication précédente.

11. Dispositif selon la revendication 10, incluant en outre un circuit électrique en connexion avec un ampèremètre ou un voltmètre pour détecter le changement de conductivité de la pâte formant le matériau capteur.

12. Dispositif selon la revendication 10, dans lequel les première et seconde électrodes sont :
a) situées sur un substrat rigide ; ou
b) situées sur un substrat souple ; ou
c) font partie d'un circuit complexe.

13. Dispositif selon la revendication 10, dans lequel :
a) le substrat rigide selon la revendication 12a est sélectionné parmi du verre, un matériau polymère et une carte de circuit imprimé ; ou
b) le substrat souple selon la revendication 12b est du papier ou un matériau polymère ; ou
c) le circuit complexe selon la revendication 12c est une puce de communication en champ proche (NFC) ou une puce d'identification par radiofréquences (RFID).

14. Procédé de détection d'un analyte comprenant :
la fourniture d'un dispositif de détection selon l'une quelconque des revendications 10 à 13 ;
l'exposition du matériau capteur à un environnement, dans lequel un changement de la conductivité du matériau capteur indique la présence de l'analyte ; et
la détection dudit changement de conductivité du matériau capteur.

15. Procédé selon la revendication 14, comprenant en outre :
a) la transmission des changements détectés de conductivité sans fil à un autre dispositif pour analyse et stockage ;
b) la détection de l'analyte par l'intermédiaire d'une communication de radiofréquences sans fil ; et
c) la détection d'une sortie de l'étiquette d'identification de radiofréquences incluant le capteur.

16. Procédé selon la revendication 14, dans lequel l'analyte est :
a) une vapeur ; ou
b) sélectionné dans le groupe consistant en un thiol, un ester, un aldéhyde, un alcool, un éther, un alcène, un alcyne, une cétone, un acide, une base ou une combinaison de ceux-ci ; ou
c) une moisissure ; ou
d) de l'éthylène, un gaz contenant de l'azote ou une amine ; ou
e) de la putrescine ou cadavérine ; ou
f) dans la plage de 0 à 10 %, 0 à 5 %, 0 à 1%, 0 à 1000 ppm, 0 à 100 ppm, 0 à 80 ppm, 0 à 50 ppm, 0 à 10 ppm, 0 à 5 ppm, 0 à 1 ppm, 0 à 0,5 ppm, 0 à 100 ppb, 0 à 50 ppb, ou 0 à 10 ppb.

17. Procédé selon la revendication 14, dans lequel le matériau capteur subit en outre :
a) un changement volumétrique lors d'une interaction avec l'analyte ; et
la détection du changement volumétrique et la dérivation d'informations relatives à l'analyte à partir dudit changement volumétrique ; ou
b) un changement de couleur lors d'une interaction avec l'analyte ; et
la détection du changement de couleur et la dérivation d'informations relatives à l'analyte à partir dudit changement de couleur.

18. Procédé selon la revendication 14, dans lequel l'analyte est en interaction avec le détecteur :
a) pour former une interaction Van der Waals, une liaison covalente, une liaison ionique, une liaison d'hydrogène ou une liaison dative ; ou
b) via un événement de liaison entre des paires de molécules biologiques, dans lequel les molécules biologiques sont des protéines, acides nucléiques, glycoprotéines, carbohydrates ou hormones.
